# EUROPEAN PATENT APPLICATION

(11) **EP 3 904 381 A1**
(43) Date of publication of application: **03.11.2021**
(21) Application number: 19901455.6
(22) Date of filing: 26.12.2019
(51) Int. Cl.: C07K 16/18, C07K 16/46, A61K 39/00, C07K 19/00

(54) **ANTIBODY FUSION PROTEIN, PREPARATION METHOD THEREFOR AND APPLICATION THEREOF**

(30) Priority: 28.12.2018 CN 201811620872
(71) Applicant: Changchun Genescience Pharmaceutical Co., Ltd., Changchun, Jilin 130012 (CN)
(72) Inventor: FENG, Xiao, Changchun, Jilin 130012 (CN); WANG, Tao, Changchun, Jilin 130012 (CN); JIN, Lei, Changchun, Jilin 130012 (CN); GUO, Hongrui, Changchun, Jilin 130012 (CN); LIU, Shuang, Changchun, Jilin 130012 (CN); HAN, Ning, Changchun, Jilin 130012 (CN); LIANG, Yangqiu, Changchun, Jilin 130012 (CN); CHEN, Yuheng, Changchun, Jilin 130012 (CN)
(74) Representative: HGF
(86) International application number: PCT/CN2019/128585
(87) International publication number: WO 2020/135556

(57) **Abstract**

Provided are an antibody fusion protein, a preparation method thereof and an application thereof. The antibody fusion protein is high in expression quantity, and the transient expression quantity in mammalian cells 293E is 100-150 mg/L; the antibody fusion protein is high in assembly rate, and the correct assembly rate exceeds 95%; the antibody fusion protein has a high affinity, and a single-sided antibody/fusion protein and antigen binding KD value is equivalent to a positive control monoclonal antibody/fusion protein and antigen binding KD value; the antibody fusion protein is convenient to purify, and the purity can reach more than 95% in one-step purification by using Protein A or Protein L, and the tumor inhibition rate in a pharmacodynamic experiment animal can reach up to 92%.

SEQUENCE LISTING

<110>  CHANGCHUN GENESCIENCE PHARMACEUTICALS CO., LTD.

<120>  ANTIBODY FUSION PROTEIN, PREPARATION METHOD THEREFOR AND APPLICATION
THEREOF

<130>  PCT/CN2019/128585

<150>  CN 201811620872.2
<151>  2018-12-28

<150>  PCT/CN2019/128585
<151>  2019-12-26

<160>  13

<170>  SIPOSequenceListing 1.0

<210>  1
<211>  205
<212>  PRT
<213>  Artificial Sequence

<220>
<223>  Trap binding to human VEGF-A

<400>  1
Ser Asp Thr Gly Arg Pro Phe Val Glu Met Tyr Ser Glu Ile Pro Glu
1               5                   10                  15
Ile Ile His Met Thr Glu Gly Arg Glu Leu Val Ile Pro Cys Arg Val
            20                  25                  30
Thr Ser Pro Asn Ile Thr Val Thr Leu Lys Lys Phe Pro Leu Asp Thr
        35                  40                  45
Leu Ile Pro Asp Gly Lys Arg Ile Ile Trp Asp Ser Arg Lys Gly Phe
    50                  55                  60
Ile Ile Ser Asn Ala Thr Tyr Lys Glu Ile Gly Leu Leu Thr Cys Glu
65                  70                  75                  80
Ala Thr Val Asn Gly His Leu Tyr Lys Thr Asn Tyr Leu Thr His Arg
                85                  90                  95
Gln Thr Asn Thr Ile Ile Asp Val Val Leu Ser Pro Ser His Gly Ile
            100                 105                 110
Glu Leu Ser Val Gly Glu Lys Leu Val Leu Asn Cys Thr Ala Arg Thr
        115                 120                 125
Glu Leu Asn Val Gly Ile Asp Phe Asn Trp Glu Tyr Pro Ser Ser Lys
    130                 135                 140
His Gln His Lys Lys Leu Val Asn Arg Asp Leu Lys Thr Gln Ser Gly
145                 150                 155                 160
Ser Glu Met Lys Lys Phe Leu Ser Thr Leu Thr Ile Asp Gly Val Thr
                165                 170                 175
Arg Ser Asp Gln Gly Leu Tyr Thr Cys Ala Ala Ser Ser Gly Leu Met
            180                 185                 190
Thr Lys Lys Asn Ser Thr Phe Val Arg Val His Glu Lys
        195                 200                 205

<210>  2
<211>  107
<212>  PRT
<213>  Artificial Sequence

<220>
<223>  VL of 047 Ab-6

<400>  2
Asp Ile Gln Met Thr Gln Ser Pro Ser Ser Leu Ser Ala Ser Val Gly
1               5                   10                  15
Asp Arg Val Thr Ile Thr Cys Ser Ala Ser Gln Asp Val Thr Thr Ala
            20                  25                  30
Val Ala Trp Tyr Gln Gln Lys Pro Gly Lys Ala Pro Lys Leu Leu Ile
        35                  40                  45
Tyr Ser Ala Ser Phe Pro Tyr Arg Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Tyr Met Tyr His Pro Ser
                85                  90                  95
Thr Phe Gly Gln Gly Thr Lys Val Glu Ile Lys
            100                 105

<210>  3
<211>  118
<212>  PRT
<213>  Artificial Sequence

<220>
<223>  VH of 047 Ab-6

<400>  3
Glu Val Gln Leu Val Glu Ser Gly Gly Gly Leu Val Gln Pro Gly Gly
1               5                   10                  15
Ser Leu Arg Leu Ser Cys Ala Ala Ser Tyr Phe Pro Phe Ser Asp Ser
            20                  25                  30
Trp Ile His Trp Val Arg Gln Ala Pro Gly Lys Gly Leu Glu Trp Val
        35                  40                  45
Ala Trp Ile Ser Pro Tyr Gly Gly Ser Ser Tyr Tyr Ala Asp Ser Val
    50                  55                  60
Gln Asp Arg Phe Thr Ile Ser Ala Asp Thr Ser Lys Asn Thr Ala Tyr
65                  70                  75                  80
Leu Gln Met Asn Ser Leu Arg Ala Glu Asp Thr Ala Val Tyr Tyr Cys
                85                  90                  95
Ala Lys Arg His Trp Pro Gly Gly Phe Asp His Trp Gly Gln Gly Thr
            100                 105                 110
Leu Val Thr Val Ser Ser
        115

<210>  4
<211>  443
<212>  PRT
<213>  Artificial Sequence

<220>
<223>  H1 heavy chain

<400>  4
Glu Val Gln Leu Val Glu Ser Gly Gly Gly Leu Val Gln Pro Gly Gly
1               5                   10                  15
Ser Leu Arg Leu Ser Cys Ala Ala Ser Tyr Phe Pro Phe Ser Asp Ser
            20                  25                  30
Trp Ile His Trp Val Arg Gln Ala Pro Gly Lys Gly Leu Glu Trp Val
        35                  40                  45
Ala Trp Ile Ser Pro Tyr Gly Gly Ser Ser Tyr Tyr Ala Asp Ser Val
    50                  55                  60
Gln Asp Arg Phe Thr Ile Ser Ala Asp Thr Ser Lys Asn Thr Ala Tyr
65                  70                  75                  80
Leu Gln Met Asn Ser Leu Arg Ala Glu Asp Thr Ala Val Tyr Tyr Cys
                85                  90                  95

Ala Lys Arg His Trp Pro Gly Gly Phe Asp His Trp Gly Gln Gly Thr
            100                 105                 110
Leu Val Thr Val Ser Ser Ala Ser Thr Lys Gly Pro Ser Val Phe Pro
        115                 120                 125
Leu Ala Pro Ser Ser Lys Ser Thr Ser Gly Gly Thr Ala Ala Leu Gly
    130                 135                 140
Cys Leu Val Lys Asp Tyr Phe Pro Glu Pro Val Thr Val Ser Trp Asn
145                 150                 155                 160
Ser Gly Ala Leu Thr Ser Gly Val His Thr Phe Pro Ala Val Leu Gln
                165                 170                 175
Ser Ser Gly Leu Tyr Ser Leu Ser Ser Val Val Thr Val Pro Ser Ser
            180                 185                 190
Ser Leu Gly Thr Gln Thr Tyr Ile Cys Asn Val Asn His Lys Pro Ser
        195                 200                 205
Asn Thr Lys Val Asp Lys Lys Val Glu Pro Lys Ser Cys Asp Lys Gly
    210                 215                 220
Gly Gly Gly Ser Gly Gly Gly Gly Ser Gly Gly Gly Gly Ser Ser Asp
225                 230                 235                 240
Thr Gly Arg Pro Phe Val Glu Met Tyr Ser Glu Ile Pro Glu Ile Ile
                245                 250                 255
His Met Thr Glu Gly Arg Glu Leu Val Ile Pro Cys Arg Val Thr Ser
            260                 265                 270
Pro Asn Ile Thr Val Thr Leu Lys Lys Phe Pro Leu Asp Thr Leu Ile
        275                 280                 285
Pro Asp Gly Lys Arg Ile Ile Trp Asp Ser Arg Lys Gly Phe Ile Ile
    290                 295                 300
Ser Asn Ala Thr Tyr Lys Glu Ile Gly Leu Leu Thr Cys Glu Ala Thr
305                 310                 315                 320
Val Asn Gly His Leu Tyr Lys Thr Asn Tyr Leu Thr His Arg Gln Thr
                325                 330                 335
Asn Thr Ile Ile Asp Val Val Leu Ser Pro Ser His Gly Ile Glu Leu
            340                 345                 350
Ser Val Gly Glu Lys Leu Val Leu Asn Cys Thr Ala Arg Thr Glu Leu
        355                 360                 365
Asn Val Gly Ile Asp Phe Asn Trp Glu Tyr Pro Ser Ser Lys His Gln
    370                 375                 380
His Lys Lys Leu Val Asn Arg Asp Leu Lys Thr Gln Ser Gly Ser Glu
385                 390                 395                 400
Met Lys Lys Phe Leu Ser Thr Leu Thr Ile Asp Gly Val Thr Arg Ser
                405                 410                 415
Asp Gln Gly Leu Tyr Thr Cys Ala Ala Ser Ser Gly Leu Met Thr Lys
            420                 425                 430
Lys Asn Ser Thr Phe Val Arg Val His Glu Lys
        435                 440

<210>  5
<211>  666
<212>  PRT
<213>  Artificial Sequence

<220>
<223>  H2 heavy chain

<400>  5
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asn Tyr
            20                  25                  30
Val Met His Trp Val Arg Gln Ala Pro Gly Gln Arg Leu Glu Trp Met
        35                  40                  45
Gly Tyr Ile Asn Pro Tyr Asn Asp Gly Thr Lys Tyr Ser Glu Lys Phe
    50                  55                  60
Lys Gly Arg Val Thr Ile Thr Arg Asp Thr Ser Ala Ser Thr Ala Tyr

65                  70                  75                  80
Met Glu Leu Ser Ser Leu Arg Ser Glu Asp Thr Ala Val Tyr Tyr Cys
                85                  90                  95
Ala Arg Gly Gly Leu Phe Thr Phe Asp Asn Trp Gly Gln Gly Thr Leu
            100                 105                 110
Val Thr Val Ser Ser Ala Ser Thr Lys Gly Pro Ser Val Phe Pro Leu
        115                 120                 125
Ala Pro Ser Ser Lys Ser Thr Ser Gly Gly Thr Ala Ala Leu Gly Cys
    130                 135                 140
Leu Val Lys Asp Tyr Phe Pro Glu Pro Val Thr Val Ser Trp Asn Ser
145                 150                 155                 160
Gly Ala Leu Thr Ser Gly Val His Thr Phe Pro Ala Val Leu Gln Ser
                165                 170                 175
Ser Gly Leu Tyr Ser Leu Ser Ser Val Val Thr Val Pro Ser Ser Ser
            180                 185                 190
Leu Gly Thr Gln Thr Tyr Ile Cys Asn Val Asn His Lys Pro Ser Asn
        195                 200                 205
Thr Lys Val Asp Lys Lys Val Glu Pro Lys Ser Cys Gly Gly Gly Gly
    210                 215                 220
Ser Gly Gly Gly Gly Ser Gly Gly Gly Gly Ser Ser Asp Thr Gly Arg
225                 230                 235                 240
Pro Phe Val Glu Met Tyr Ser Glu Ile Pro Glu Ile Ile His Met Thr
                245                 250                 255
Glu Gly Arg Glu Leu Val Ile Pro Cys Arg Val Thr Ser Pro Asn Ile
            260                 265                 270
Thr Val Thr Leu Lys Lys Phe Pro Leu Asp Thr Leu Ile Pro Asp Gly
        275                 280                 285
Lys Arg Ile Ile Trp Asp Ser Arg Lys Gly Phe Ile Ile Ser Asn Ala
    290                 295                 300
Thr Tyr Lys Glu Ile Gly Leu Leu Thr Cys Glu Ala Thr Val Asn Gly
305                 310                 315                 320
His Leu Tyr Lys Thr Asn Tyr Leu Thr His Arg Gln Thr Asn Thr Ile
                325                 330                 335
Ile Asp Val Val Leu Ser Pro Ser His Gly Ile Glu Leu Ser Val Gly
            340                 345                 350
Glu Lys Leu Val Leu Asn Cys Thr Ala Arg Thr Glu Leu Asn Val Gly
        355                 360                 365
Ile Asp Phe Asn Trp Glu Tyr Pro Ser Ser Lys His Gln His Lys Lys
    370                 375                 380
Leu Val Asn Arg Asp Leu Lys Thr Gln Ser Gly Ser Glu Met Lys Lys
385                 390                 395                 400
Phe Leu Ser Thr Leu Thr Ile Asp Gly Val Thr Arg Ser Asp Gln Gly
                405                 410                 415
Leu Tyr Thr Cys Ala Ala Ser Ser Gly Leu Met Thr Lys Lys Asn Ser
            420                 425                 430
Thr Phe Val Arg Val His Glu Lys Asp Lys Thr His Thr Cys Pro Pro
        435                 440                 445
Cys Pro Ala Pro Glu Leu Leu Gly Gly Pro Ser Val Phe Leu Phe Pro
    450                 455                 460
Pro Lys Pro Lys Asp Thr Leu Met Ile Ser Arg Thr Pro Glu Val Thr
465                 470                 475                 480
Cys Val Val Val Asp Val Ser His Glu Asp Pro Glu Val Lys Phe Asn
                485                 490                 495
Trp Tyr Val Asp Gly Val Glu Val His Asn Ala Lys Thr Lys Pro Arg
            500                 505                 510
Glu Glu Gln Tyr Asn Ser Thr Tyr Arg Val Val Ser Val Leu Thr Val
        515                 520                 525
Leu His Gln Asp Trp Leu Asn Gly Lys Glu Tyr Lys Cys Lys Val Ser
    530                 535                 540
Asn Lys Ala Leu Pro Ala Pro Ile Glu Lys Thr Ile Ser Lys Ala Lys
545                 550                 555                 560
Gly Gln Pro Arg Glu Pro Gln Val Tyr Thr Leu Pro Pro Ser Arg Asp
                565                 570                 575

Glu Leu Thr Lys Asn Gln Val Ser Leu Thr Cys Leu Val Lys Gly Phe
            580                 585                 590
Tyr Pro Ser Asp Ile Ala Val Glu Trp Glu Ser Asn Gly Gln Pro Glu
        595                 600                 605
Asn Asn Tyr Lys Thr Thr Pro Pro Val Leu Asp Ser Asp Gly Ser Phe
    610                 615                 620
Phe Leu Tyr Ser Lys Leu Thr Val Asp Lys Ser Arg Trp Gln Gln Gly
625                 630                 635                 640
Asn Val Phe Ser Cys Ser Val Met His Glu Ala Leu His Asn His Tyr
                645                 650                 655
Thr Gln Lys Ser Leu Ser Leu Ser Pro Gly
            660                 665

<210> 6
<211> 666
<212> PRT
<213> Artificial Sequence

<220>
<223> H3 heavy chain

<400> 6
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asn Tyr
            20                  25                  30
Val Met His Trp Val Arg Gln Ala Pro Gly Gln Arg Leu Glu Trp Met
        35                  40                  45
Gly Tyr Ile Asn Pro Tyr Asn Asp Gly Thr Lys Tyr Ser Glu Lys Phe
    50                  55                  60
Lys Gly Arg Val Thr Ile Thr Arg Asp Thr Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Leu Phe Thr Phe Asp Asn Trp Gly Gln Gly Thr Leu
            100                 105                 110
Val Thr Val Ser Ser Ala Ser Thr Lys Gly Pro Ser Val Phe Pro Leu
        115                 120                 125
Ala Pro Ser Ser Lys Ser Thr Ser Gly Gly Thr Ala Ala Leu Gly Cys
    130                 135                 140
Leu Val Lys Asp Tyr Phe Pro Glu Pro Val Thr Val Ser Trp Asn Ser
145                 150                 155                 160
Gly Ala Leu Thr Ser Gly Val His Thr Phe Pro Ala Val Leu Gln Ser
                165                 170                 175
Ser Gly Leu Tyr Ser Leu Ser Ser Val Val Thr Val Pro Ser Ser Ser
            180                 185                 190
Leu Gly Thr Gln Thr Tyr Ile Cys Asn Val Asn His Lys Pro Ser Asn
        195                 200                 205
Thr Lys Val Asp Lys Lys Val Glu Pro Lys Ser Cys Asp Lys Thr His
    210                 215                 220
Thr Cys Pro Pro Cys Pro Ala Pro Glu Leu Leu Gly Gly Pro Ser Val
225                 230                 235                 240
Phe Leu Phe Pro Pro Lys Pro Lys Asp Thr Leu Met Ile Ser Arg Thr
                245                 250                 255
Pro Glu Val Thr Cys Val Val Val Asp Val Ser His Glu Asp Pro Glu
            260                 265                 270
Val Lys Phe Asn Trp Tyr Val Asp Gly Val Glu Val His Asn Ala Lys
        275                 280                 285
Thr Lys Pro Arg Glu Glu Gln Tyr Asn Ser Thr Tyr Arg Val Val Ser
    290                 295                 300
Val Leu Thr Val Leu His Gln Asp Trp Leu Asn Gly Lys Glu Tyr Lys
305                 310                 315                 320
Cys Lys Val Ser Asn Lys Ala Leu Pro Ala Pro Ile Glu Lys Thr Ile

325                             330                             335
Ser Lys Ala Lys Gly Gln Pro Arg Glu Pro Gln Val Tyr Thr Leu Pro
                340                             345                             350
Pro Ser Arg Asp Glu Leu Thr Lys Asn Gln Val Ser Leu Thr Cys Leu
        355                             360                             365
Val Lys Gly Phe Tyr Pro Ser Asp Ile Ala Val Glu Trp Glu Ser Asn
    370                             375                             380
Gly Gln Pro Glu Asn Asn Tyr Lys Thr Thr Pro Pro Val Leu Asp Ser
385                             390                             395                             400
Asp Gly Ser Phe Phe Leu Tyr Ser Lys Leu Thr Val Asp Lys Ser Arg
                        405                             410                             415
Trp Gln Gln Gly Asn Val Phe Ser Cys Ser Val Met His Glu Ala Leu
                420                             425                             430
His Asn His Tyr Thr Gln Lys Ser Leu Ser Leu Ser Pro Gly Gly Gly
        435                             440                             445
Gly Gly Ser Gly Gly Gly Gly Ser Gly Gly Gly Gly Ser Ser Asp Thr
    450                             455                             460
Gly Arg Pro Phe Val Glu Met Tyr Ser Glu Ile Pro Glu Ile Ile His
465                             470                             475                             480
Met Thr Glu Gly Arg Glu Leu Val Ile Pro Cys Arg Val Thr Ser Pro
                        485                             490                             495
Asn Ile Thr Val Thr Leu Lys Lys Phe Pro Leu Asp Thr Leu Ile Pro
                500                             505                             510
Asp Gly Lys Arg Ile Ile Trp Asp Ser Arg Lys Gly Phe Ile Ile Ser
        515                             520                             525
Asn Ala Thr Tyr Lys Glu Ile Gly Leu Leu Thr Cys Glu Ala Thr Val
    530                             535                             540
Asn Gly His Leu Tyr Lys Thr Asn Tyr Leu Thr His Arg Gln Thr Asn
545                             550                             555                             560
Thr Ile Ile Asp Val Val Leu Ser Pro Ser His Gly Ile Glu Leu Ser
                        565                             570                             575
Val Gly Glu Lys Leu Val Leu Asn Cys Thr Ala Arg Thr Glu Leu Asn
                580                             585                             590
Val Gly Ile Asp Phe Asn Trp Glu Tyr Pro Ser Ser Lys His Gln His
        595                             600                             605
Lys Lys Leu Val Asn Arg Asp Leu Lys Thr Gln Ser Gly Ser Glu Met
    610                             615                             620
Lys Lys Phe Leu Ser Thr Leu Thr Ile Asp Gly Val Thr Arg Ser Asp
625                             630                             635                             640
Gln Gly Leu Tyr Thr Cys Ala Ala Ser Ser Gly Leu Met Thr Lys Lys
                        645                             650                             655
Asn Ser Thr Phe Val Arg Val His Glu Lys
                660                             665

<210>  7
<211>  563
<212>  PRT
<213>  Artificial Sequence

<220>
<223>  H4 heavy chain

<400>  7
Gln Val Gln Leu Val Gln Ser Gly Ala Glu Val Lys Lys Pro Gly Ala
1                               5                               10                              15
Ser Val Lys Val Ser Cys Lys Ala Ser Gly Tyr Thr Phe Thr Asn Tyr
                20                              25                              30
Val Met His Trp Val Arg Gln Ala Pro Gly Gln Arg Leu Glu Trp Met
        35                              40                              45
Gly Tyr Ile Asn Pro Tyr Asn Asp Gly Thr Lys Tyr Ser Glu Lys Phe
    50                              55                              60
Lys Gly Arg Val Thr Ile Thr Arg Asp Thr Ser Ala Ser Thr Ala Tyr
65                              70                              75                              80

Met Glu Leu Ser Ser Leu Arg Ser Glu Asp Thr Ala Val Tyr Tyr Cys
                85                  90                  95
Ala Arg Gly Gly Leu Phe Thr Phe Asp Asn Trp Gly Gln Gly Thr Leu
            100                 105                 110
Val Thr Val Ser Ser Gly Gly Gly Gly Ser Gly Gly Gly Gly Ser Gly
        115                 120                 125
Gly Gly Gly Ser Ser Asp Thr Gly Arg Pro Phe Val Glu Met Tyr Ser
    130                 135                 140
Glu Ile Pro Glu Ile Ile His Met Thr Glu Gly Arg Glu Leu Val Ile
145                 150                 155                 160
Pro Cys Arg Val Thr Ser Pro Asn Ile Thr Val Thr Leu Lys Lys Phe
                165                 170                 175
Pro Leu Asp Thr Leu Ile Pro Asp Gly Lys Arg Ile Ile Trp Asp Ser
            180                 185                 190
Arg Lys Gly Phe Ile Ile Ser Asn Ala Thr Tyr Lys Glu Ile Gly Leu
        195                 200                 205
Leu Thr Cys Glu Ala Thr Val Asn Gly His Leu Tyr Lys Thr Asn Tyr
    210                 215                 220
Leu Thr His Arg Gln Thr Asn Thr Ile Ile Asp Val Val Leu Ser Pro
225                 230                 235                 240
Ser His Gly Ile Glu Leu Ser Val Gly Glu Lys Leu Val Leu Asn Cys
                245                 250                 255
Thr Ala Arg Thr Glu Leu Asn Val Gly Ile Asp Phe Asn Trp Glu Tyr
            260                 265                 270
Pro Ser Ser Lys His Gln His Lys Lys Leu Val Asn Arg Asp Leu Lys
        275                 280                 285
Thr Gln Ser Gly Ser Glu Met Lys Lys Phe Leu Ser Thr Leu Thr Ile
    290                 295                 300
Asp Gly Val Thr Arg Ser Asp Gln Gly Leu Tyr Thr Cys Ala Ala Ser
305                 310                 315                 320
Ser Gly Leu Met Thr Lys Lys Asn Ser Thr Phe Val Arg Val His Glu
                325                 330                 335
Lys Asp Lys Thr His Thr Cys Pro Pro Cys Pro Ala Pro Glu Leu Leu
            340                 345                 350
Gly Gly Pro Ser Val Phe Leu Phe Pro Pro Lys Pro Lys Asp Thr Leu
        355                 360                 365
Met Ile Ser Arg Thr Pro Glu Val Thr Cys Val Val Val Asp Val Ser
    370                 375                 380
His Glu Asp Pro Glu Val Lys Phe Asn Trp Tyr Val Asp Gly Val Glu
385                 390                 395                 400
Val His Asn Ala Lys Thr Lys Pro Arg Glu Glu Gln Tyr Asn Ser Thr
                405                 410                 415
Tyr Arg Val Val Ser Val Leu Thr Val Leu His Gln Asp Trp Leu Asn
            420                 425                 430
Gly Lys Glu Tyr Lys Cys Lys Val Ser Asn Lys Ala Leu Pro Ala Pro
        435                 440                 445
Ile Glu Lys Thr Ile Ser Lys Ala Lys Gly Gln Pro Arg Glu Pro Gln
    450                 455                 460
Val Tyr Thr Leu Pro Pro Ser Arg Asp Glu Leu Thr Lys Asn Gln Val
465                 470                 475                 480
Ser Leu Ser Cys Ala Val Lys Gly Phe Tyr Pro Ser Asp Ile Ala Val
                485                 490                 495
Glu Trp Glu Ser Asn Gly Gln Pro Glu Asn Asn Tyr Lys Thr Thr Pro
            500                 505                 510
Pro Val Leu Asp Ser Asp Gly Ser Phe Phe Leu Val Ser Lys Leu Thr
        515                 520                 525
Val Asp Lys Ser Arg Trp Gln Gln Gly Asn Val Phe Ser Cys Ser Val
    530                 535                 540
Met His Glu Ala Leu His Asn His Tyr Thr Gln Lys Ser Leu Ser Leu
545                 550                 555                 560
Ser Pro Gly

<210>  8
<211>  439
<212>  PRT
<213>  Artificial Sequence

<220>
<223>  L1 light chain

<400>  8
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Val His Ser
            20                  25                  30
Lys Gly Asn Thr Tyr Leu His Trp Tyr Leu Gln Lys Pro Gly Gln Ser
        35                  40                  45
Pro Gln Leu Leu Ile Tyr Arg Val Ser Lys Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Gly Asp Val Gly Val Tyr Tyr Cys Ser Gln Ser
                85                  90                  95
Thr His Val Pro Phe Thr Phe Gly Gln Gly Thr Lys Val Glu Ile Lys
            100                 105                 110
Arg Thr Val Ala Ala Pro Ser Val Phe Ile Phe Pro Pro Ser Asp Glu
        115                 120                 125
Gln Leu Lys Ser Gly Thr Ala Ser Val Val Cys Leu Leu Asn Asn Phe
    130                 135                 140
Tyr Pro Arg Glu Ala Lys Val Gln Trp Lys Val Asp Asn Ala Leu Gln
145                 150                 155                 160
Ser Gly Asn Ser Gln Glu Ser Val Thr Glu Gln Asp Ser Lys Asp Ser
                165                 170                 175
Thr Tyr Ser Leu Ser Ser Thr Leu Thr Leu Ser Lys Ala Asp Tyr Glu
            180                 185                 190
Lys His Lys Val Tyr Ala Cys Glu Val Thr His Gln Gly Leu Ser Ser
        195                 200                 205
Pro Val Thr Lys Ser Phe Asn Arg Gly Glu Cys Gly Gly Gly Gly Ser
    210                 215                 220
Gly Gly Gly Gly Ser Gly Gly Gly Gly Ser Ser Asp Thr Gly Arg Pro
225                 230                 235                 240
Phe Val Glu Met Tyr Ser Glu Ile Pro Glu Ile Ile His Met Thr Glu
                245                 250                 255
Gly Arg Glu Leu Val Ile Pro Cys Arg Val Thr Ser Pro Asn Ile Thr
            260                 265                 270
Val Thr Leu Lys Lys Phe Pro Leu Asp Thr Leu Ile Pro Asp Gly Lys
        275                 280                 285
Arg Ile Ile Trp Asp Ser Arg Lys Gly Phe Ile Ile Ser Asn Ala Thr
    290                 295                 300
Tyr Lys Glu Ile Gly Leu Leu Thr Cys Glu Ala Thr Val Asn Gly His
305                 310                 315                 320
Leu Tyr Lys Thr Asn Tyr Leu Thr His Arg Gln Thr Asn Thr Ile Ile
                325                 330                 335
Asp Val Val Leu Ser Pro Ser His Gly Ile Glu Leu Ser Val Gly Glu
            340                 345                 350
Lys Leu Val Leu Asn Cys Thr Ala Arg Thr Glu Leu Asn Val Gly Ile
        355                 360                 365
Asp Phe Asn Trp Glu Tyr Pro Ser Ser Lys His Gln His Lys Lys Leu
    370                 375                 380
Val Asn Arg Asp Leu Lys Thr Gln Ser Gly Ser Glu Met Lys Lys Phe
385                 390                 395                 400
Leu Ser Thr Leu Thr Ile Asp Gly Val Thr Arg Ser Asp Gln Gly Leu
                405                 410                 415
Tyr Thr Cys Ala Ala Ser Ser Gly Leu Met Thr Lys Lys Asn Ser Thr
            420                 425                 430

Phe Val Arg Val His Glu Lys
        435

<210>  9
<211>  219
<212>  PRT
<213>  Artificial Sequence

<220>
<223>  L2 light chain

<400>  9
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Val His Ser
            20                  25                  30
Lys Gly Asn Thr Tyr Leu His Trp Tyr Leu Gln Lys Pro Gly Gln Ser
        35                  40                  45
Pro Gln Leu Leu Ile Tyr Arg Val Ser Lys Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Gly Asp Val Gly Val Tyr Tyr Cys Ser Gln Ser
                85                  90                  95
Thr His Val Pro Phe Thr Phe Gly Gln Gly Thr Lys Val Glu Ile Lys
            100                 105                 110
Arg Thr Val Ala Ala Pro Ser Val Phe Ile Phe Pro Pro Ser Asp Glu
        115                 120                 125
Gln Leu Lys Ser Gly Thr Ala Ser Val Val Cys Leu Leu Asn Asn Phe
    130                 135                 140
Tyr Pro Arg Glu Ala Lys Val Gln Trp Lys Val Asp Asn Ala Leu Gln
145                 150                 155                 160
Ser Gly Asn Ser Gln Glu Ser Val Thr Glu Gln Asp Ser Lys Asp Ser
                165                 170                 175
Thr Tyr Ser Leu Ser Ser Thr Leu Thr Leu Ser Lys Ala Asp Tyr Glu
            180                 185                 190
Lys His Lys Val Tyr Ala Cys Glu Val Thr His Gln Gly Leu Ser Ser
        195                 200                 205
Pro Val Thr Lys Ser Phe Asn Arg Gly Glu Cys
    210                 215

<210>  10
<211>  558
<212>  PRT
<213>  Artificial Sequence

<220>
<223>  L3 light chain

<400>  10
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Val His Ser
            20                  25                  30
Lys Gly Asn Thr Tyr Leu His Trp Tyr Leu Gln Lys Pro Gly Gln Ser
        35                  40                  45
Pro Gln Leu Leu Ile Tyr Arg Val Ser Lys Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Gly Asp Val Gly Val Tyr Tyr Cys Ser Gln Ser
                85                  90                  95
Thr His Val Pro Phe Thr Phe Gly Gln Gly Thr Lys Val Glu Ile Lys

                    100                 105                 110
Gly Gly Gly Gly Ser Gly Gly Gly Gly Ser Gly Gly Gly Gly Ser Ser
        115                 120                 125
Asp Thr Gly Arg Pro Phe Val Glu Met Tyr Ser Glu Ile Pro Glu Ile
    130                 135                 140
Ile His Met Thr Glu Gly Arg Glu Leu Val Ile Pro Cys Arg Val Thr
145                 150                 155                 160
Ser Pro Asn Ile Thr Val Thr Leu Lys Lys Phe Pro Leu Asp Thr Leu
                165                 170                 175
Ile Pro Asp Gly Lys Arg Ile Ile Trp Asp Ser Arg Lys Gly Phe Ile
            180                 185                 190
Ile Ser Asn Ala Thr Tyr Lys Glu Ile Gly Leu Leu Thr Cys Glu Ala
        195                 200                 205
Thr Val Asn Gly His Leu Tyr Lys Thr Asn Tyr Leu Thr His Arg Gln
    210                 215                 220
Thr Asn Thr Ile Ile Asp Val Val Leu Ser Pro Ser His Gly Ile Glu
225                 230                 235                 240
Leu Ser Val Gly Glu Lys Leu Val Leu Asn Cys Thr Ala Arg Thr Glu
                245                 250                 255
Leu Asn Val Gly Ile Asp Phe Asn Trp Glu Tyr Pro Ser Ser Lys His
            260                 265                 270
Gln His Lys Lys Leu Val Asn Arg Asp Leu Lys Thr Gln Ser Gly Ser
        275                 280                 285
Glu Met Lys Lys Phe Leu Ser Thr Leu Thr Ile Asp Gly Val Thr Arg
    290                 295                 300
Ser Asp Gln Gly Leu Tyr Thr Cys Ala Ala Ser Ser Gly Leu Met Thr
305                 310                 315                 320
Lys Lys Asn Ser Thr Phe Val Arg Val His Glu Lys Asp Lys Thr His
                325                 330                 335
Thr Cys Pro Pro Cys Pro Ala Pro Glu Leu Leu Gly Gly Pro Ser Val
            340                 345                 350
Phe Leu Phe Pro Pro Lys Pro Lys Asp Thr Leu Met Ile Ser Arg Thr
        355                 360                 365
Pro Glu Val Thr Cys Val Val Val Asp Val Ser His Glu Asp Pro Glu
    370                 375                 380
Val Lys Phe Asn Trp Tyr Val Asp Gly Val Glu Val His Asn Ala Lys
385                 390                 395                 400
Thr Lys Pro Arg Glu Glu Gln Tyr Asn Ser Thr Tyr Arg Val Val Ser
                405                 410                 415
Val Leu Thr Val Leu His Gln Asp Trp Leu Asn Gly Lys Glu Tyr Lys
            420                 425                 430
Cys Lys Val Ser Asn Lys Ala Leu Pro Ala Pro Ile Glu Lys Thr Ile
        435                 440                 445
Ser Lys Ala Lys Gly Gln Pro Arg Glu Pro Gln Val Tyr Thr Leu Pro
    450                 455                 460
Pro Ser Arg Asp Glu Leu Thr Lys Asn Gln Val Ser Leu Trp Cys Leu
465                 470                 475                 480
Val Lys Gly Phe Tyr Pro Ser Asp Ile Ala Val Glu Trp Glu Ser Asn
                485                 490                 495
Gly Gln Pro Glu Asn Asn Tyr Lys Thr Thr Pro Pro Val Leu Asp Ser
            500                 505                 510
Asp Gly Ser Phe Phe Leu Tyr Ser Lys Leu Thr Val Asp Lys Ser Arg
        515                 520                 525
Trp Gln Gln Gly Asn Val Phe Ser Cys Ser Val Met His Glu Ala Leu
    530                 535                 540
His Asn His Tyr Thr Gln Lys Ser Leu Ser Leu Ser Pro Gly
545                 550                 555

<210>  11
<211>  234
<212>  PRT
<213>  Artificial Sequence

<220>
<223>  Human PD-L1-His

<400>  11
Phe Thr Val Thr Val Pro Lys Asp Leu Tyr Val Val Glu Tyr Gly Ser
1               5                   10                  15
Asn Met Thr Ile Glu Cys Lys Phe Pro Val Glu Lys Gln Leu Asp Leu
            20                  25                  30
Ala Ala Leu Ile Val Tyr Trp Glu Met Glu Asp Lys Asn Ile Ile Gln
        35                  40                  45
Phe Val His Gly Glu Glu Asp Leu Lys Val Gln His Ser Ser Tyr Arg
    50                  55                  60
Gln Arg Ala Arg Leu Leu Lys Asp Gln Leu Ser Leu Gly Asn Ala Ala
65                  70                  75                  80
Leu Gln Ile Thr Asp Val Lys Leu Gln Asp Ala Gly Val Tyr Arg Cys
                85                  90                  95
Met Ile Ser Tyr Gly Gly Ala Asp Tyr Lys Arg Ile Thr Val Lys Val
            100                 105                 110
Asn Ala Pro Tyr Asn Lys Ile Asn Gln Arg Ile Leu Val Val Asp Pro
        115                 120                 125
Val Thr Ser Glu His Glu Leu Thr Cys Gln Ala Glu Gly Tyr Pro Lys
    130                 135                 140
Ala Glu Val Ile Trp Thr Ser Ser Asp His Gln Val Leu Ser Gly Lys
145                 150                 155                 160
Thr Thr Thr Thr Asn Ser Lys Arg Glu Glu Lys Leu Phe Asn Val Thr
                165                 170                 175
Ser Thr Leu Arg Ile Asn Thr Thr Thr Asn Glu Ile Phe Tyr Cys Thr
            180                 185                 190
Phe Arg Arg Leu Asp Pro Glu Glu Asn His Thr Ala Glu Leu Val Ile
        195                 200                 205
Pro Glu Leu Pro Leu Ala His Pro Pro Asn Glu Arg Thr Ser Gly Gly
    210                 215                 220
Gly Gly Ser His His His His His His
225                 230

<210>  12
<211>  354
<212>  PRT
<213>  Artificial Sequence

<220>
<223>  Human VEGF-A-mFc

<400>  12
Ala Pro Met Ala Glu Gly Gly Gly Gln Asn His His Glu Val Val Lys
1               5                   10                  15
Phe Met Asp Val Tyr Gln Arg Ser Tyr Cys His Pro Ile Glu Thr Leu
            20                  25                  30
Val Asp Ile Phe Gln Glu Tyr Pro Asp Glu Ile Glu Tyr Ile Phe Lys
        35                  40                  45
Pro Ser Cys Val Pro Leu Met Arg Cys Gly Gly Cys Cys Asn Asp Glu
    50                  55                  60
Gly Leu Glu Cys Val Pro Thr Glu Glu Ser Asn Ile Thr Met Gln Ile
65                  70                  75                  80
Met Arg Ile Lys Pro His Gln Gly Gln His Ile Gly Glu Met Ser Phe
                85                  90                  95
Leu Gln His Asn Lys Cys Glu Cys Arg Pro Lys Lys Asp Arg Ala Arg
            100                 105                 110
Gln Glu Lys Cys Asp Lys Pro Arg Arg Ser Gly Gly Gly Gly Ser Val
        115                 120                 125
Pro Arg Asp Cys Gly Cys Lys Pro Cys Ile Cys Thr Val Pro Glu Val
    130                 135                 140
Ser Ser Val Phe Ile Phe Pro Pro Lys Pro Lys Asp Val Leu Thr Ile

145                 150                 155                 160
Thr Leu Thr Pro Lys Val Thr Cys Val Val Val Asp Ile Ser Lys Asp
                165                 170                 175
Asp Pro Glu Val Gln Phe Ser Trp Phe Val Asp Asp Val Glu Val His
            180                 185                 190
Thr Ala Gln Thr Gln Pro Arg Glu Glu Gln Phe Asn Ser Thr Phe Arg
        195                 200                 205
Ser Val Ser Glu Leu Pro Ile Met His Gln Asp Trp Leu Asn Gly Lys
    210                 215                 220
Glu Phe Lys Cys Arg Val Asn Ser Ala Ala Phe Pro Ala Pro Ile Glu
225                 230                 235                 240
Lys Thr Ile Ser Lys Thr Lys Gly Arg Pro Lys Ala Pro Gln Val Tyr
                245                 250                 255
Thr Ile Pro Pro Pro Lys Glu Gln Met Ala Lys Asp Lys Val Ser Leu
            260                 265                 270
Thr Cys Met Ile Thr Asp Phe Phe Pro Glu Asp Ile Thr Val Glu Trp
        275                 280                 285
Gln Trp Asn Gly Gln Pro Ala Glu Asn Tyr Lys Asn Thr Gln Pro Ile
    290                 295                 300
Met Asp Thr Asp Gly Ser Tyr Phe Val Tyr Ser Lys Leu Asn Val Gln
305                 310                 315                 320
Lys Ser Asn Trp Glu Ala Gly Asn Thr Phe Thr Cys Ser Val Leu His
                325                 330                 335
Glu Gly Leu His Asn His His Thr Glu Lys Ser Leu Ser His Ser Pro
            340                 345                 350
Gly Lys


<210>  13
<211>  134
<212>  PRT
<213>  Artificial Sequence

<220>
<223>  Human VEGF-A-His

<400>  13
Ala Pro Met Ala Glu Gly Gly Gly Gln Asn His His Glu Val Val Lys
1               5                   10                  15
Phe Met Asp Val Tyr Gln Arg Ser Tyr Cys His Pro Ile Glu Thr Leu
            20                  25                  30
Val Asp Ile Phe Gln Glu Tyr Pro Asp Glu Ile Glu Tyr Ile Phe Lys
        35                  40                  45
Pro Ser Cys Val Pro Leu Met Arg Cys Gly Gly Cys Cys Asn Asp Glu
    50                  55                  60
Gly Leu Glu Cys Val Pro Thr Glu Glu Ser Asn Ile Thr Met Gln Ile
65                  70                  75                  80
Met Arg Ile Lys Pro His Gln Gly Gln His Ile Gly Glu Met Ser Phe
                85                  90                  95
Leu Gln His Asn Lys Cys Glu Cys Arg Pro Lys Lys Asp Arg Ala Arg
            100                 105                 110
Gln Glu Lys Cys Asp Lys Pro Arg Arg Ser Gly Gly Gly Gly Ser His
        115                 120                 125
His His His His His His
    130

## Description

### CROSS REFERENCE TO RELATED APPLICATIONS

This application claims the priority of Chinese Patent Application No. 201811620872.2, filed to China National Intellectual Property Administration on December 28, 2018, and titled with "ANTIBODY FUSION PROTEIN, PREPARATION METHOD THEREFOR AND APPLICATION THEREOF", and the disclosures of which are hereby incorporated by reference.

### FIELD

The present disclosure relates to the field of medicine, specifically to an antibody fusion protein, preparation method thereof and application thereof.

### BACKGROUND

A bispecific monoclonal antibody (BsAb) is a special antibody that is artificially made to bind two different antigens at the same time. Bispecific antibodies can recognize both tumor target cells and immune effector cells, so they have dual functions of antibody specificity and mediating the cytotoxicity of effector cells. Bispecific antibodies can recruit effector cells at tumor sites and activate effector cells to exert anti-tumor effects. The mechanism of killing tumor cells mediated by bispecific antibodies includes cell proliferation, cytokine release, cytotoxic peptides and regulation of enzymes. *In vivo* and clinical studies have proved that bispecific antibody-mediated immunotherapy can treat tumors in some animals, and clinically can mitigate the condition of patients with tumor and prolong their life. Therefore, the application of bispecific antibody-mediated immunocompetent cells in tumor therapy has a good prospect.

Bispecific antibodies are not nature products and can only be prepared artificially. Bi- or multi-specific antibodies in the art can bind to at least two antigens and can be produced by cell fusion, chemical conjugation or recombinant DNA technology. Recently, a wide variety of recombinant bispecific antibody structures have been developed, such as tetravalent bispecific antibodies by fusion of, for example, an IgG antibody and a single-chain domain (Coloma, M.J., et al., Nature Biotech. 15 (1997) 159-163; WO2001077342; and Morrison, S.L., Nature Biotech. 25 (2007) 1233-1234). In addition, many other new forms that can bind to more than two antigens have been developed, in which the main structure of the antibody (IgA, IgD, IgE, IgG or IgM) is no longer limited to, such as diabodies, triabodies or tetrabodies, minibodies and several single-chain forms (scFv, Bis-scFv) (Holliger, P., et al., Nature Biotech. 23 (2005) 1126-1136; Fischer, N., and Léger, O., Pathobiology 74 (2007) 3-14; Shen, J., et al., Journal of Immunogical Methods 318 (2007) 65-74; Wu, C., et al., Nature Biotech. 25 (2007) 1290-1297).

In one method, the cell quadroma technology (Milstein, C. and A. C. Cuello, Nature, 305 (1983) 537-40) is utilized to produce a bispecific antibody that is very similar to a natural antibody. The cell quadroma technology is based on the somatic fusion of two different hybridoma cell lines expressing murine monoclonal antibodies with the desired bispecific antibody specificity. Because of the random pairing of two different heavy and light chains of antibodies in the hybridoma cell lines, up to 10 different antibody types will be generated, of which only one is the desired functional bispecific antibody. Due to the presence of mismatched by-products and significantly low yields, complicated purification procedures are required (Morrison, S.L., Nature Biotech 25 (2007) 1233-1234). Generally, if recombinant expression technology is used, the same problem of mismatch by-products still exists.

A method used to avoid the problem of mismatch by-products is called "knobs-into-holes". The purpose is to force the heavy chains from two different antibodies to pair with each other by introducing mutation into the CH3 domain to modify the contact interface. In one chain, amino acids with large volume are replaced by amino acids with short side chains to form a "hole". Conversely, amino acids with a large side chain are introduced to the other CH3 domain to form a "knob". By co-expressing these two heavy chains, a higher yield of heterodimer form ("knob-hole") compared with homodimer form ("hole-hole" or "knob-knob") was observed (Ridgway, J.B., Presta, L.G., Carter, P. and WO 1996027011). The percentage of heterodimer can be further increased by reconstruction of the interaction interface of the two CH3 domains using phage display method and introduction of disulfide bonds to stabilize the heterodimer (Merchant, A.M., et al., Nature Biotech 16 (1998) 677-681; Atwell, S., Ridgway, J.B., Wells, J.A., Carter, P., J. Mol. Biol. 270 (1997) 26-35). An important constraint of this strategy is that the light chains of the two parent antibodies must be the same to prevent mismatches and formation of inactive molecules.

In addition to the "knob-hole" structure, the Fc pairing of different half-antibodies can also be achieved through the strand-exchange engineered domain (SEED) technology of IgG and IgA CH3 (Davis, J.H., et al., Protein Eng. Des. Sel., 2010, 23(4): 195-202.).

In order to solve the problem of the incorrect assembly of different light chains, a new process of double-cell line expressing half-antibodies separately and *in vitro* assembly has been developed recently. Inspired by the half-antibody random exchange process of human IgG4 antibodies naturally occurring under physiological conditions, GenMab has developed FAE (Fab-arm exchange) bifunctional antibody technology (Gramer, M.J., et al., MAbs 2013, 5(6): 962-973.). Introducing two point mutations, K409R and F405L, into the IgG1 heavy chain CH3 domains of the two target antibodies respectively, can produce half-antibody exchange rearrangement similar to that of IgG4 antibodies. Two different IgG1 antibodies after mutation were expressed in two CHO cell lines respectively, and the assembly between the light and heavy chains of each half-antibody was completed. After protein A affinity purification, precise assembly between heterogeneous half-antibodies can be achieved *in vitro* by using a mild oxidant system.

In addition to sharing light chains with the same sequence or performing *in vitro* assembly, the correct assembly of light chains of antibodies can also be facilitated by Crossmab technology. A representative product is Roche's Ang-2/VEGF CrossMab CH1-CL. Based on the modification of "knobs-into-holes", Crossmab technology exchanged CL and CH1 in the Fab domain of Ang-2 antibody and remained the Fab domain of VEGF antibody unchanged. The light chain of the modified Ang-2 antibody is not easily mismatched with the heavy chain of the VEGF antibody, and the "knob-hole" structure can promote the heterodimerization of the two heavy chains (Schaefer, W, et al., Proc Natl. Acad. Sci. USA, 2011, 108(27): 11187-11192.).

Moreover, two single-chain antibodies (scFv) or two Fabs can be linked through a peptide to form a bifunctional antibody fragment. A representative product is BiTE (bispecific T-cell engager) series products developed by Micromet in German. This series of products is generated by linking anti-CD3 single-chain antibodies with the single-chain antibodies against different anti-tumor cell surface antigens through a peptide (Baeuerle, P.A., et al., Cancer Res., 2009, 69(12): 4941-4944). The advantage of such antibody structure is that it has a small molecular weight, can be expressed in prokaryotic cells, and does not require the consideration of incorrect assembly; while the disadvantage is that it cannot mediate some corresponding biological functions due to a lack of antibody Fc fragment, and its half-life is short.

In addition, the related bispecific antibody proteins in the prior art also have the disadvantages of low expression levels in transient transfection and low affinity, and the complexity of some purification processes, which makes it difficult to meet the needs of industrial production.

### SUMMARY

In view of the above, the present disclosure provides an antibody fusion protein, preparation method thereof and application thereof. The bispecific antibody fusion protein has advantages of high expression level, high assembly rate, high affinity, and easiness of purification. The purity of one-step purification using Protein A or Protein L can reach more than 95%.

In order to achieve the above objects of the present disclosure, the present disclosure provides the following technical solutions.

The present disclosure provides an antibody fusion protein, comprising
(I) an antibody that specifically binds to a first antigen,
(II) a flexible peptide, and
(III) a fusion protein that specifically binds to a second antigen.

In some specific embodiments of the present disclosure, the antibody comprises one or more fragments selected from light chain variable region (VL), light chain constant region (CL), heavy chain variable region (VH), heavy chain constant region 1 (CH1), heavy chain constant region 2 (CH2), and heavy chain constant region 3 (CH3).

In some specific embodiments of the present disclosure, the antibody further comprises a hinge region.

In some specific embodiments of the present disclosure, the antibody fusion protein comprises
a1) light chain variable region and light chain constant region of the antibody that specifically binds to the first antigen, the flexible peptide and the fusion protein that specifically binds to the second antigen, represented as VL-CL-Linker-Trap, and
b1) heavy chain variable region, heavy chain constant region 1 and partial hinge region of the antibody that specifically binds to the first antigen, the flexible peptide, and the fusion protein that specifically binds to the second antigen, represented as VH-CH1-Partial hinge-Linker- Trap;
or comprises
a2) light chain of the antibody that specifically binds to the first antigen, and
b2) heavy chain variable region and heavy chain constant region 1 of the antibody that specifically binds to the first antigen, the flexible peptide, the fusion protein that specifically binds to the second antigen, heavy chain constant region 2 and heavy chain constant region 3, represented as VH-CH1-Linker-Trap-CH2-CH3;
or comprises
a3) light chain of the antibody that specifically binds to the first antigen, and
b3) heavy chain variable region, heavy chain constant region 1, heavy chain constant region 2 and heavy chain constant region 3 of the antibody that specifically binds to the first antigen, the flexible peptide, and the fusion protein that specifically binds to the second antigen, represented as VH-CH1-CH2-CH3-Linker-Trap;
or comprises
a4) light chain variable region of the antibody that specifically binds to the first antigen, the flexible peptide, the fusion protein that specifically binds to the second antigen, heavy chain constant region 2 and heavy chain constant region 3, represented as VL-Linker-Trap-CH2-CH3, and
b4) heavy chain variable region of the antibody that specifically binds to the first antigen, the flexible peptide, the fusion protein that specifically binds to the second antigen, heavy chain constant region 2 and heavy chain constant region 3, represented as VH-Linker-Trap-CH2-CH3.

In some specific embodiments of the present disclosure, the flexible peptide comprises a sequence of (G4S)ₙ, wherein n is an integer greater than 0; preferably an integer of 1-10.

In some specific embodiments of the present disclosure, the light chain constant region (CL) and the heavy chain constant region 1 (CH1) form a heterodimer, and the terminal cysteine residue in the light chain constant region (CL) and the cysteine residue in the hinge region of heavy chain form a disulfide bond.

In some embodiments of the present disclosure, the cysteine residues in the hinge regions of heavy chains form a disulfide bond.

In some specific embodiments of the present disclosure, the domain of the heavy chain constant region 3 (CH3) of first heavy chain and the domain of the heavy chain constant region 3 (CH3) of second heavy chain are modified to a structure that facilitates the formation of the antibody fusion protein.

In some specific embodiments of the present disclosure, the modification comprises
c) modification to the domain of the heavy chain constant region 3 (CH3) of the first heavy chain: in the interface between the domain of the heavy chain constant region 3 (CH3) of the first heavy chain and the domain of the heavy chain constant region 3 (CH3) of the second heavy chain of bivalent bispecific antibody, an amino acid residue in the domain of the heavy chain constant region 3 (CH3) of the first heavy chain is replaced with an amino acid residue with a volume larger than the original amino acid residue to form a knob in the domain of the heavy chain constant region 3 (CH3) of the first heavy chain, wherein the knob is capable of inserting into a hole of the domain of the heavy chain constant region 3 (CH3) of the second heavy chain, and
d) modification to the domain of the heavy chain constant region 3 (CH3) of the second heavy chain: in the interface between the domain of the heavy chain constant region 3 (CH3) of the second heavy chain and the domain of the heavy chain constant region 3 (CH3) of the first heavy chain of bivalent bispecific antibody, an amino acid residue in the domain of the heavy chain constant region 3 (CH3) of the second heavy chain is replaced with an amino acid residue with a volume smaller than the original amino acid residue to form a hole in the domain of the heavy chain constant region 3 (CH3) of the second heavy chain, wherein the hole is capable of holding the knob of the domain of the heavy chain constant region 3 (CH3) of the first heavy chain.

In some specific embodiments of the present disclosure, wherein in the heavy chain,

The amino acid residue with a volume larger than the original amino acid residue is selected from the group consisting of arginine, phenylalanine, tyrosine, and tryptophan; and

The amino acid residue with a volume smaller than the original amino acid residue is selected from the group consisting of alanine, serine, threonine, and valine.

In some specific embodiments of the present disclosure,
(IV) the heavy chain with an amino acid sequence as shown in SEQ ID NO: 4, SEQ ID NO: 5, SEQ ID NO: 6 or SEQ ID NO: 7, and
(V) the light chain with an amino acid sequence as shown in SEQ ID NO: 8, SEQ ID NO: 9 or SEQ ID NO: 10;
or (VI) an amino acid sequence derived from the amino acid sequence described in (IV) or (V) by substitution, deletion or addition of one or more amino acids, and functionally identical or similar to the amino acid sequence described in (IV) or (V);
or (VII) an amino acid sequence with more than 90% homology with the sequence described in (IV) or (V);
or (VIII) an amino acid sequence that has the same functional fragment or functional variant as the sequence described in (IV) or (V);
wherein the antibody fusion protein specifically binds to hPD-L1 and hVEGF-A; and

The first antigen is hPD-L1 and the second antigen is hVEGF-A.

In some specific embodiments of the present disclosure, wherein the one or more amino acids is 2-10 amino acids.

In some specific embodiments of the present disclosure, the antibody fusion protein comprises
(IX) a heavy chain with an amino acid sequence of SEQ ID NO: 4, and a light chain with an amino acid sequence of SEQ ID NO: 8; or
(X) a heavy chain with an amino acid sequence of SEQ ID NO: 5, and a light chain with an amino acid sequence of SEQ ID NO: 9; or
(XI) a heavy chain with an amino acid sequence of SEQ ID NO: 6, and a light chain with an amino acid sequence of SEQ ID NO: 9; or
(XII) a heavy chain with an amino acid sequence of SEQ ID NO: 7, and a light chain with an amino acid sequence of SEQ ID NO: 10.

In some specific embodiments of the present disclosure, the antibody fusion protein specifically binds to hPD-L1 and hVEGF-A, wherein the first antigen is hPD-L1, and the second antigen is hVEGF-A.

In some specific embodiments of the present disclosure, the antibody fusion protein with FabT structure has an amino acid sequence of heavy chain as shown in SEQ ID NO: 4, and an amino acid sequence of light chain as shown in SEQ ID NO: 8; the antibody fusion protein with FTF structure has an amino acid sequence of heavy chain as shown in SEQ ID NO: 5, and an amino acid sequence of light chain as shown in SEQ ID NO: 9; the antibody fusion protein with IgGT structure has an amino acid sequence of heavy chain as shown in SEQ ID NO: 6, and an amino acid sequence of light chain as shown in SEQ ID NO: 9; and the antibody fusion protein with FvT structure has an amino acid sequence of heavy chain as shown in SEQ ID NO: 7, and an amino acid sequence of light chain as shown in SEQ ID NO: 10.

The present disclosure also provides a nucleic acid molecule encoding the antibody fusion protein, comprising
(XIII) a nucleic acid encoding the heavy chain variable region as shown in SEQ ID NO: 4, SEQ ID NO: 5, SEQ ID NO: 6 or SEQ ID NO: 7, and a nucleic acid encoding the light chain variable region as shown in SEQ ID NO: 8, SEQ ID NO: 9 or SEQ ID NO: 10; or
(XIV) a nucleic acid having complementary sequence of the heavy chain variable region as shown in SEQ ID NO: 4, SEQ ID NO: 5, SEQ ID NO: 6 or SEQ ID NO: 7, and a nucleic acid having complementary sequence of the light chain variable region as shown in SEQ ID NO: 8, SEQ ID NO: 9 or SEQ ID NO: 10; or
(XV) a nucleotide sequence encoding the same protein as the nucleotide sequence described in (XIII) or (XIV), but different from the nucleotide sequence described in (XIII) or (XIV) due to the degeneracy of genetic code; or
(XVI) a sequence with more than 90% homology with the sequence described in (XIII) or (XIV) or (XV).

In some specific embodiments of the present disclosure, the nucleic acid molecule comprises a nucleotide sequence derived from the nucleotide sequence described in (XIII) or (XIV) or (XV) or (XVI) by substitution, deletion or addition of one or more nucleotide, and functionally identical or similar to the nucleotide sequence described in (XIII) or (XIV) or (XV) or (XVI), wherein the one or more amino acids is 2-10 amino acids.

The present disclosure also provides an expression vector, comprising the nucleic acid molecule and a cell transformed with the expression vector.

The present disclosure also provides a complex comprising the antibody fusion protein covalently linked to an isotope, an immunotoxin and/or a chemical drug.

The present disclosure also provides a conjugate, formed by coupling the antibody fusion protein and/or the complex with a solid medium or a semi-solid medium.

The present disclosure also provides use of the antibody fusion protein and/or the complex and/or the conjugate for the manufacture of a medicament for the treatment of a disease and/or a composition for the diagnose of a disease; wherein the disease is selected from the group consisting of breast cancer, lung cancer, gastric cancer, intestinal cancer, esophageal cancer, ovarian cancer, cervical cancer, kidney cancer, bladder cancer, pancreatic cancer, glioma, and melanoma.

The present disclosure also provides a pharmaceutical composition comprising the antibody fusion protein and/or the complex and/or the conjugate.

The present disclosure also provides a kit comprising the antibody fusion protein and/or the complex and/or the conjugate.

The present disclosure also provides a method for treating a disease with the antibody fusion protein and/or the complex and/or the conjugate, wherein the disease is selected from the group consisting of breast cancer, lung cancer, gastric cancer, intestinal cancer, esophageal cancer, ovarian cancer, cervical cancer, kidney cancer, bladder cancer, pancreatic cancer, non-Hodgkin's lymphoma, chronic lymphoma leukemia, multiple myeloma, acute myeloid leukemia, acute lymphoma leukemia, glioma, melanoma, diabetic macular edema, and wet macular degeneration.

The present disclosure also provides a method for producing the antibody fusion protein, comprising transforming a host cell with the expression vector, culturing the host cell under conditions that allow the synthesis of the antibody fusion protein, and recovering the antibody fusion protein from the culture.

The antibody fusion proteins provided by the present disclosure have a high expression level with transient expression of 100-150 mg/L in mammalian cell 293E; a high assembly rate with a correct assembly rate of more than 95%; a high affinity with a binding KD value of single-sided antibody/fusion protein to the antigen comparable to that of the positive control monoclonal antibody/fusion protein to the antigen; and easiness of purification with a purity of one-step purification using Protein A or Protein L up to more than 95%.

### BRIEF DESCRIPTION OF DRAWINGS

In order to illustrate examples of the present disclosure or technical solutions in the prior art more clearly, drawings required to be used in the description of the examples or prior art will be introduced briefly below.
FIG. 1 shows a schematic diagram of the bispecific antibody fusion protein with FabT structure.
FIG. 2 shows a schematic diagram of the bispecific antibody fusion protein with FTF structure.
FIG. 3 shows a schematic diagram of the bispecific antibody fusion protein with IgGT structure.
FIG. 4 shows a schematic diagram of the bispecific antibody fusion protein with FvT structure.
FIG. 5 shows the SDS-PAGE results of the transient expression of bispecific antibody fusion proteins with FabT, FTF, IgGT and FvT structures. M indicates Maker; Lanes 1 and 2 represent the non-reducing electrophoresis and reducing electrophoresis of FabT, respectively; Lanes 3 and 4 represent the non-reducing electrophoresis and reducing electrophoresis of FTF, respectively; Lanes 5 and 6 represent the non-reducing electrophoresis and reducing electrophoresis of IgGT, respectively; and Lanes 7 and 8 represent the non-reducing electrophoresis and reducing electrophoresis of FvT, respectively.
FIG. 6A and FIG. 6B show the ELISA results of the supernatants from transient expression of bispecific antibody fusion proteins with FabT, FTF, IgGT and FvT structures.
FIG. 7 shows the SDS-PAGE results of bispecific antibody fusion proteins with FabT, FTF, IgGT and FvT structures after purification. M indicates Marker; Lanes 1 and 2 represent the non-reducing electrophoresis of FabT purified by Protein L; Lanes 2 and 4 represent the reducing electrophoresis of FTF purified by Mabselect Sure; Lanes 5 and 6 represent the non-reducing electrophoresis of IgGT purified by Mabselect Sure; and Lanes 7 and 8 represent the reducing electrophoresis of FvT purified by Mabselect Sure.
FIG. 8 shows the ELISA results of bispecific antibody fusion proteins with FabT, FTF, IgGT and FvT structures after purification.
FIG. 9 shows the detection results of the activity of bispecific antibody fusion proteins with FabT, FTF, IgGT and FvT structures blocking the cellular VEGFA signaling pathway.
FIG. 10 shows the detection results of T cell activation activity of bispecific antibody fusion proteins with FabT, FTF, IgGT and FvT structures.

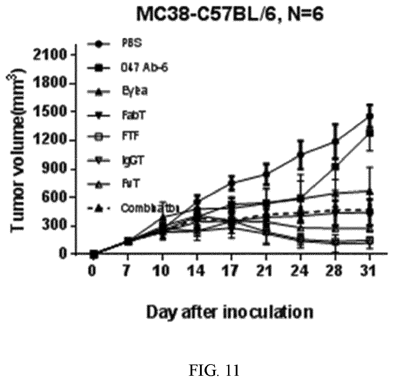

FIG. 11 shows the results of animal pharmacodynamic experiments of bispecific antibody fusion proteins with FabT, FTF, IgGT and FvT structures.

### DETAILED DESCRIPTION

The present disclosure discloses an antibody fusion protein, a preparation method thereof and application thereof. In view of the content herein, those skilled in the art can make appropriate modifications to the process parameters. It should be particularly indicated that, all similar replacements and changes are obvious for those skilled in the art, which are deemed to be included in the present disclosure. The methods and uses of the present disclosure have been described by way of preferred embodiments, and it will be apparent to those skilled in the art that changes as well as appropriate modifications and combinations of the methods and uses described herein may be made without departing from the content, spirit and scope of the present disclosure, to achieve and apply the techniques of the present disclosure.

The bispecific antibody fusion protein of the present disclosure comprises
a). light chain variable region and light chain constant region of the antibody that specifically binds to the first antigen, the flexible peptide and the fusion protein that specifically binds to the second antigen, represented as VL-CL-Linker-Trap, and
b). heavy chain variable region, heavy chain constant region 1 and partial hinge region of the antibody that specifically binds to the first antigen, the flexible peptide, and the fusion protein that specifically binds to the second antigen, represented as VH-CH1-Partial hinge-Linker-Trap; or
c). light chain of the antibody that specifically binds to the first antigen, and
d). heavy chain variable region and heavy chain constant region 1 of the antibody that specifically binds to the first antigen, the flexible peptide, the fusion protein that specifically binds to the second antigen, heavy chain constant region 2 and heavy chain constant region 3, represented as VH-CH1-Linker-Trap-CH2-CH3; or
e). light chain of the antibody that specifically binds to the first antigen, and
f). heavy chain variable region, heavy chain constant region 1, heavy chain constant region 2 and heavy chain constant region 3 of the antibody that specifically binds to the first antigen, the flexible peptide, and the fusion protein that specifically binds to the second antigen, represented as VH-CH1-CH2-CH3-Linker-Trap; or
g). light chain variable region of the antibody that specifically binds to the first antigen, the flexible peptide, the fusion protein that specifically binds to the second antigen, heavy chain constant region 2 and heavy chain constant region 3, represented as VL-Linker-Trap-CH2-CH3, and
h). heavy chain variable region of the antibody that specifically binds to the first antigen, the flexible peptide, the fusion protein that specifically binds to the second antigen, heavy chain constant region 2 and heavy chain constant region 3, represented as VH-Linker-Trap-CH2-CH3.

Further, the flexible peptide comprises a sequence of (G4S)ₙ, wherein n is an integer greater than 0.

Further, CL and CH1 form a heterodimer, and the terminal cysteine residue in CL and the cysteine residue in the hinge region of heavy chain form a disulfide bond; or
further, the cysteine residues in the hinge regions of heavy chains form a disulfide bond.

Further, the CH3 domain of first heavy chain and the CH3 domain of second heavy chain are modified to a structure that facilitates the formation of the antibody fusion protein.

The bispecific antibody fusion protein can be modified, and the modification comprises
a) modification to the CH3 domain of the first heavy chain: in the interface between the CH3 domain of the first heavy chain and the CH3 domain of the second heavy chain of bivalent bispecific antibody, an amino acid residue in the CH3 domain of the first heavy chain is replaced with an amino acid residue with a volume larger than the original amino acid residue to form a knob in the CH3 domain of the first heavy chain, wherein the knob is capable of inserting into a hole of the CH3 domain of the second heavy chain, and
b) modification to the CH3 domain of the second heavy chain: in the interface between the CH3 domain of the second heavy chain and the CH3 domain of the first heavy chain of bivalent bispecific antibody, an amino acid residue in the CH3 domain of the second heavy chain is replaced with an amino acid residue with a volume smaller than the original amino acid residue to form a hole in the CH3 domain of the second heavy chain, wherein the hole is capable of holding the knob of the CH3 domain of the first heavy chain.

The amino acid residues with a volume larger than the original amino acid residue is selected from the group consisting of arginine, phenylalanine, tyrosine, and tryptophan.

The amino acid residues with a volume smaller than the original amino acid residue is selected from the group consisting of alanine, serine, threonine, and valine.

Further, the bispecific antibody fusion protein is a bispecific antibody that specifically binds to hPD-L1 and hVEGF-A. The bispecific antibody fusion protein with FabT structure has an amino acid sequence of heavy chain as shown in SEQ ID NO: 4, and an amino acid sequence of light chain as shown in SEQ ID NO: 8; the bispecific antibody fusion protein with FTF structure has an amino acid sequence of heavy chain as shown in SEQ ID NO: 5, and an amino acid sequence of light chain as shown in SEQ ID NO: 9; the bispecific antibody fusion protein with IgGT structure has an amino acid sequence of heavy chain as shown in SEQ ID NO: 6, and an amino acid sequence of light chain as shown in SEQ ID NO: 9; and the bispecific antibody fusion protein with FvT structure has an amino acid sequence of heavy chain as shown in SEQ ID NO: 7, and an amino acid sequence of light chain as shown in SEQ ID NO: 10.

The method for producing the bispecific antibody fusion proteins of the present disclosure comprises the following steps:
a) transforming a host cell with
   a vector comprising a nucleic acid molecule encoding light chain variable region and light chain constant region of the antibody that specifically binds to the first antigen, the flexible peptide and the fusion protein that specifically binds to the second antigen, and
   a vector comprising a nucleic acid molecule encoding heavy chain variable region, heavy chain constant region 1 and partial hinge region of the antibody that specifically binds to the first antigen, the flexible peptide, and the fusion protein that specifically binds to the second antigen; or
   a vector comprising a nucleic acid molecule encoding light chain of the antibody that specifically binds to the first antigen, and
   a vector comprising a nucleic acid molecule encoding heavy chain variable region and heavy chain constant region 1 of the antibody that specifically binds to the first antigen, the flexible peptide, the fusion protein that specifically binds to the second antigen, heavy chain constant region 2 and heavy chain constant region 3; or
   a vector comprising a nucleic acid molecule encoding light chain of the antibody that specifically binds to the first antigen, and
   a vector comprising a nucleic acid molecule encoding heavy chain variable region, heavy chain constant region 1, heavy chain constant region 2 and heavy chain constant region 3 of the antibody that specifically binds to the first antigen, the flexible peptide, and the fusion protein that specifically binds to the second antigen; or
   a vector comprising a nucleic acid molecule encoding light chain variable region of the antibody that specifically binds to the first antigen, the flexible peptide, the fusion protein that specifically binds to the second antigen, heavy chain constant region 2 and heavy chain constant region 3, and
   a vector comprising a nucleic acid molecule encoding heavy chain variable region of the antibody that specifically binds to the first antigen, the flexible peptide, the fusion protein that specifically binds to the second antigen, heavy chain constant region 2 and heavy chain constant region 3;
b) culturing the host cell under conditions that allow the synthesis of the bispecific antibody fusion protein; and
c) recovering the antibody fusion protein from the culture.

The host cell of the present disclosure comprises
a vector comprising a nucleic acid molecule encoding light chain variable region and light chain constant region of the antibody that specifically binds to the first antigen, the flexible peptide and the fusion protein that specifically binds to the second antigen, and
a vector comprising a nucleic acid molecule encoding heavy chain variable region, heavy chain constant region 1 and partial hinge region of the antibody that specifically binds to the first antigen, the flexible peptide, and the fusion protein that specifically binds to the second antigen; or
a vector comprising a nucleic acid molecule encoding light chain of the antibody that specifically binds to the first antigen, and
a vector comprising a nucleic acid molecule encoding heavy chain variable region and heavy chain constant region 1 of the antibody that specifically binds to the first antigen, the flexible peptide, the fusion protein that specifically binds to the second antigen, heavy chain constant region 2 and heavy chain constant region 3; or
a vector comprising a nucleic acid molecule encoding light chain of the antibody that specifically binds to the first antigen, and
a vector comprising a nucleic acid molecule encoding heavy chain variable region, heavy chain constant region 1, heavy chain constant region 2 and heavy chain constant region 3 of the antibody that specifically binds to the first antigen, the flexible peptide, and the fusion protein that specifically binds to the second antigen; or
a vector comprising a nucleic acid molecule encoding light chain variable region of the antibody that specifically binds to the first antigen, the flexible peptide, the fusion protein that specifically binds to the second antigen, heavy chain constant region 2 and heavy chain constant region 3, and
a vector comprising a nucleic acid molecule encoding heavy chain variable region of the antibody that specifically binds to the first antigen, the flexible peptide, the fusion protein that specifically binds to the second antigen, heavy chain constant region 2 and heavy chain constant region 3.

The composition of the bispecific antibody fusion protein of the present disclosure comprises a therapeutically effective amount of any of the above bispecific antibody fusion proteins and a pharmaceutically acceptable carrier, pharmaceutically acceptable auxiliary or pharmaceutically acceptable excipient; preferably, the composition is a pharmaceutical composition (i.e., a drug) or a diagnostic composition.

Further, the pharmaceutical composition comprises any of the above bispecific antibody fusion proteins and at least one pharmaceutically acceptable excipient.

The bispecific antibody fusion protein of the present disclosure has the following excellent technical effects:
1. high expression level, the transient expression level in mammalian cells 293E is 100-150 mg/L;
2. high assembly rate, the correct assembly rate exceeds 95%;
3. high affinity, the binding KD value of single-sided antibody/fusion protein to the antigen is comparable to that of the positive control monoclonal antibody/fusion protein to the antigen;
4. simple purification process, the purity of one-step purification using Protein A or Protein L can reach more than 95%.

Raw materials, auxiliary materials and reagents used for the antibody fusion proteins, preparation method thereof and application thereof provided by the present disclosure are all purchased from the market.

The present disclosure will be further illustrated by the following examples:

### Example 1 Preparation of Bispecific Antibody Fusion Proteins

### 1. Construction of transient transfection expression vector for bispecific antibody fusion proteins Materials

The sequence of Trap (SEQ ID NO: 1) binding to human VEGF-A was derived from Regeneron's listed drug "Eylea" (for the sequence, reference could be made to the sequence listing <210> 6 of Chinese patent CN103349781B). The anti-human PD-L1 humanized monoclonal antibody was derived from 047 Ab-6 having the sequence of VL (SEQ ID NO: 2) and the sequence of VH (SEQ ID NO: 3), which was obtained by panning of the natural human source library by Genescience. Reference could be also made to Chinese patent CN201810044303.1 for the coding nucleotides of heavy chain constant region CHI, hinge region and Fc of IgG1, and nucleotides of Kappa chain constant region.

### Methods

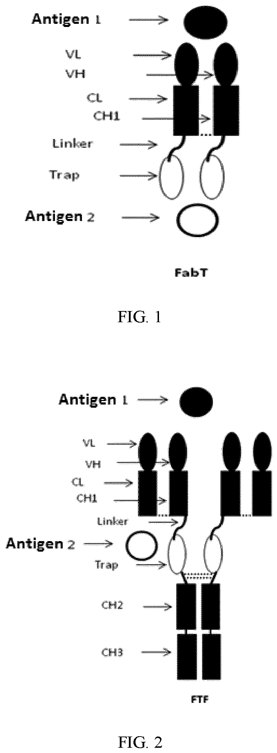

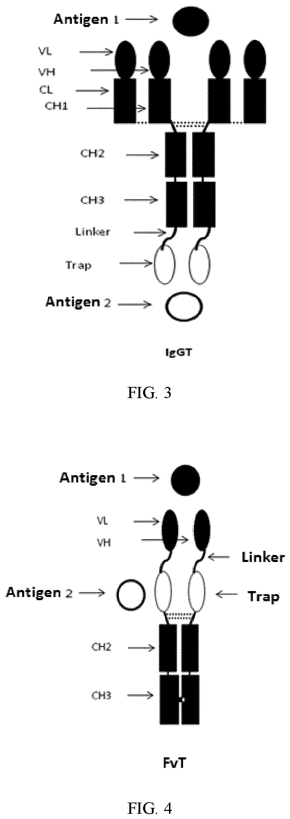

pGS003 was selected to construct the expression vectors for the heavy chain and light chain of bispecific antibody fusion proteins (4 proteins, of which the structure diagrams are shown in FIG. 1 to FIG. 4). Primers were designed according to the coding nucleotides of VEGFR1 domain 2 and VEGFR2 domain 3, the coding nucleotides of VL and VH derived from the anti-human PD-L1 humanized monoclonal antibody 047 Ab-6, the coding nucleotides of heavy chain constant region CHI, hinge region and Fc of IgG1, and nucleotide sequence of Kappa chain constant region, and multiple cloning sites in the vector. After PCR amplification, four heavy chain coding sequences and three light chain coding sequences were cloned into pGS003 by *in vitro* recombination method (Nanjing GenScript, CloneEZ PCR Cloning Kit), as shown in Table 1. After sequencing to identify the correct insertion of the target gene, the recombinant expression vectors were transformed into E. coli TOP10F'. Then a single colony was picked and inoculated in LB medium containing 100 µg/mL of ampicillin, and cultured with shaking at 37°C for 16 hours. The plasmids were extracted using endotoxin-removal, large-scale extraction kit of Zymo Research. The obtained plasmids were dissolved in 1 mL of ultrapure water, and the plasmid concentration and OD260/280 were determined with a spectrophotometer. A plasmid with OD260/280 value between 1.8 and 1.9 is considered a relatively pure plasmid DNA.

**Table 1 List of transient transfection expression vectors for heavy and light chains**

| Vector for Heavy Chain Expression | Heavy Chain Amino Acid Sequence | Vector for Light Chain Expression | Light Chain Amino Acid Sequence |
|---|---|---|---|
| H1 | SEQ ID NO: 4 | L1 | SEQ ID NO: 8 |
| H2 | SEQ ID NO: 5 | L2 | SEQ ID NO: 9 |
| H3 | SEQ ID NO: 6 | L3 | SEQ ID NO: 10 |
| H4 | SEQ ID NO: 7 | | |

### 2. Transfection, expression and detection in mammalian 293E cells

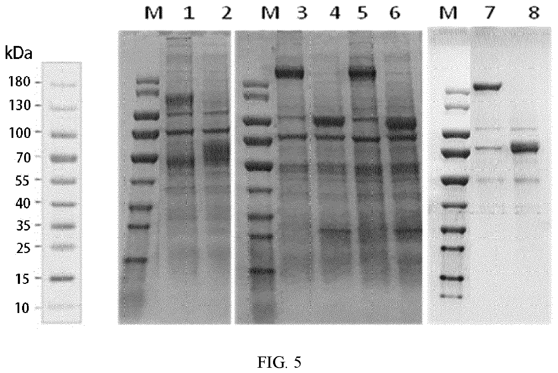

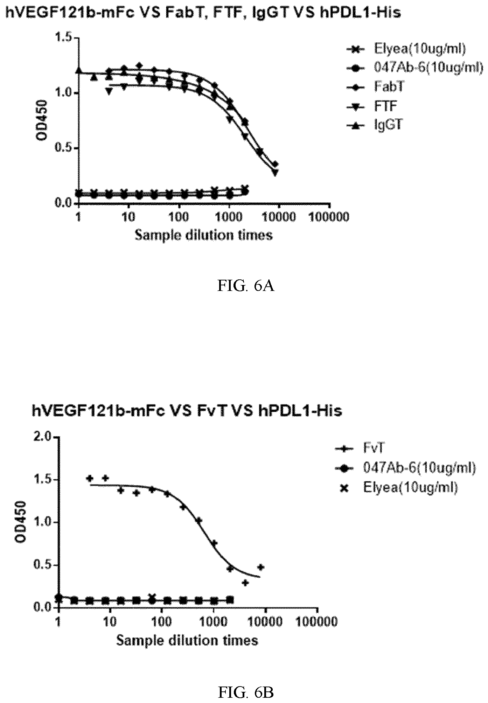

Vectors for the above four heavy chain expression vectors and three light chain expression were constructed. H1 was used to express VH of anti-hPD-L1 and the fusion protein binding hVEGF-A, L1 was used to express VL of anti-hPD-L1 and the fusion protein binding hVEGF-A, H2 was used to express VH of anti-hPD-L1 and the fusion protein binding hVEGF-A, L2 was used to express VL of anti-hPD-L1, H3 was used to express VH of anti-hPD-L1 and the fusion protein binding hVEGF-A, L3 was used to express VL of anti-hPD-L1 and the fusion protein binding hVEGF-A, and H4 was used to express VH of anti-hPD-L1 and the fusion protein binding hVEGF-A. Combinations of the above vectors, H1+L1 (FabT structure), H2+L2 (FTF structure), H3+L2 (IgGT structure), and H4+L3 (FvT structure), were subjected to transient transfection expression in 2 mL 293E system for evaluation, where the linkers in FabT, FTF, IgGT and FvT structures were all (G4S)₃. The expression levels and the ELISA detection value of the antibody binding to human VEGF-A and human PD-L1 were detected. The results are shown in FIG. 5, Table 2, Table 3, FIG. 6A and 6B. The expression, assembly and binding to antigens of FabT, FTF, IgGT and FvT structures were all fairly good.

293E cells were used to perform amplified transient transfection expression of FabT, FTF, IgGT and FvT structures in Freestyle medium. 24 hours before transfection, 300 mL of 293E cells at 0.5×10⁶ cells/mL were seeded in a 1 L cell culture flask, and cultured in a 37°C, 5% CO₂ incubator with shaking at 120 rpm. During transfection, 300 µL of 293fectin™ was added to 5.7 mL Opti-MEM™. After mixing well, the mixture was incubated at room temperature for 2 minutes. Meanwhile, 300 µg of the expression plasmids for FabT structure and FTF structure were diluted to 6 mL with OPtiMEM, respectively. The diluted transfection reagent 293 fectin and plasmids were mixed thoroughly and incubated at room temperature for 15 minutes. After that, the mixture was added to cells and mixed well, and cultured in a 37°C, 5% CO₂ incubator with shaking at 120 rpm for 7 days.

**Table 2 Transient transfection assembly rates of FabT, FTF, IgGT and FvT**

| Lane | Band | Peak | Average | Trace | Peak X Trace | Band % |
|---|---|---|---|---|---|---|
| Number | Number | Int | Int | Int x mm | | |
| 1 | 1 | 38.412 | 21.984 | 209.394 | 8043.242 | 99% |
| 1 | 2 | 8.482 | 7.951 | 8.415 | 67.44 | |
| 2 | 1 | 68.568 | 26.215 | 360.673 | 24730.626 | 99% |
| 2 | 2 | 10.163 | 8.161 | 11.517 | 82.94 | |
| 3 | 1 | 66.907 | 28.937 | 377.712 | 25271.577 | 99% |
| 3 | 2 | 12.136 | 8.815 | 12.439 | 150.959 | |
| 4 | 1 | 55.284 | 16.841 | 178.238 | 9553.71 | 99% |

**Table 3 Transient transfection expression levels of FabT, FTF, IgGT and FvT**

| Antibody structure | FabT | FTF | IgGT | FvT |
|---|---|---|---|---|
| Expression level (mg/L) | 100 | 150 | 150 | 100 |

### Example 2 Purification and Detection of Preferred Antibodies

### Purification of proteins with FabT structure

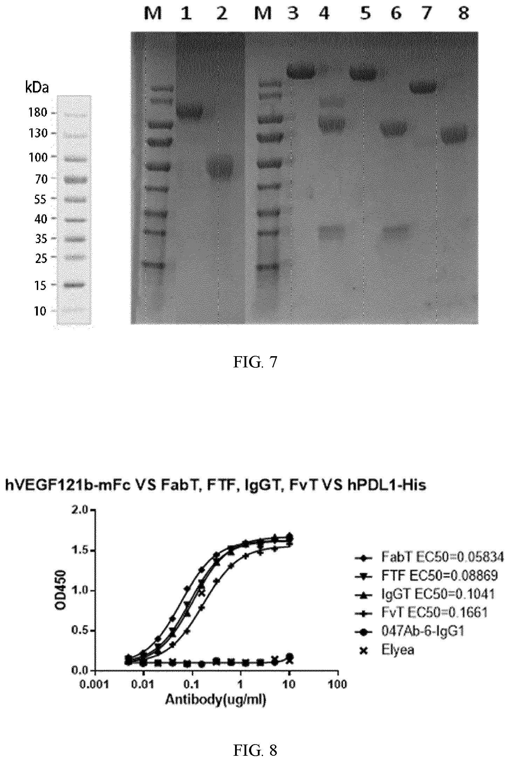

The cell culture medium was centrifuged at 2000 g for 20 min, and the supernatant was collected and then filtered with a 0.22 micron filter membrane. Next, the supernatant was subjected to Protein L (GE) chromatography, the proteins were eluted with 20 mM citrate-sodium citrate, pH 3.0, and then the resultant was adjusted to neutral pH with 1 M Tris base. Purified samples were detected by SDS-PAGE using 4-20% gradient gel (GenScript Biotechnology Co., Ltd.) to detect purified proteins. The results are shown in FIG. 7 and Table 4. The purity of FabT was 95%.

### Purification of proteins with FTF structure

The cell culture medium was centrifuged at 2000 g for 20 min, and the supernatant was collected and then filtered with a 0.22 micron filter membrane. Next, the supernatant was subjected to Mabselect Sure (GE) chromatography, the proteins were eluted with 20 mM citrate-sodium citrate, pH 3.0, and then the resultant was adjusted to neutral pH with 1 M Tris base. Purified samples were detected by SDS-PAGE using 4-20% gradient gel (GenScript Biotechnology Co., Ltd.) to detect purified proteins. The results are shown in FIG. 7 and Table 4. The purity of FTF was 95%.

### Purification of proteins with IgGT structure

The cell culture medium was centrifuged at 2000 g for 20 min, and the supernatant was collected and then filtered with a 0.22 micron filter membrane. Next, the supernatant was subjected to Mabselect Sure (GE) chromatography, the proteins were eluted with 20 mM citrate-sodium citrate, pH 3.0, and then the resultant was adjusted to neutral pH with 1 M Tris base. Purified samples were detected by SDS-PAGE using 4-20% gradient gel (GenScript Biotechnology Co., Ltd.) to detect purified proteins. The results are shown in FIG. 7 and Table 4. The purity of IgGT was 95%.

### Purification of proteins with FvT structure

The cell culture medium was centrifuged at 2000 g for 20 min, and the supernatant was collected and then filtered with a 0.22 micron filter membrane. Next, the supernatant was subjected to Mabselect Sure (GE) chromatography, the proteins were eluted with 20 mM citrate-sodium citrate, pH 3.0, and then the resultant was adjusted to neutral pH with 1 M Tris base. Purified samples were detected by SDS-PAGE using 4-20% gradient gel (GenScript Biotechnology Co., Ltd.) to detect purified proteins. The results are shown in FIG. 7 and Table 4. The purity of FvT was 95%.

**Table 4 Purities of FabT, FTF, IgGT and FvT after purification**

| Lane | Band | Peak | Average | Trace | Peak X Trace | Band % |
|---|---|---|---|---|---|---|
| Number | Number | Int | Int | Int x mm | | |
| 1 | 1 | 50.016 | 21.287 | 202.754 | 10140.944 | 99% |
| 2 | 1 | 67.747 | 27.728 | 264.105 | 17892.321 | 99% |
| 2 | 2 | 2.043 | 1.228 | 0.975 | 1.99 | |
| 3 | 1 | 58.903 | 22.344 | 212.828 | 12536.208 | 99% |
| 3 | 2 | 0.821 | 0.353 | 0.374 | 0.37 | |
| 4 | 1 | 57.728 | 25.974 | 185.551 | 10711.488 | 99% |
| 4 | 2 | 8.191 | 7.931 | 6.295 | 51.562 | |

### Example 3 ELISA Detection of Preferred Antibodies Binding to Human VEGF-A and Human PD-L1

1. Coating the first antigen: Human PD-L1-His (constructed by GeneScience, SEQ ID NO: 11) was diluted with PBS to 1 µg/mL, and then added to a 96-well microtiter plate at 50 µL per well and incubated overnight at 4°C.
2. Blocking: After being washed three times, the plate was blocked with 3% BSA at 250 µL per well, and incubated at 37°C for 2 hours.
3. Adding candidate antibody: After being washed three times, the candidate antibody was added to the plate, each with 12 samples diluted at a 2-fold concentration gradient with an initial concentration of 10 mg/mL, positive control or negative control was added at 50 µL per well, and incubated at 25°C for 1 hour.
4. Adding the second antigen: After the plate was washed three times, human VEGF-A-mFc (constructed by GeneScience, SEQ ID NO: 12) was diluted with PBS to 10 µg/mL, and then added to the 96-well microtiter plate at 50 µL per well and incubated at 25°C for 1 hour.
5. Adding the secondary antibody: After being washed three times, HRP-labeled streptavidin (1:10,000) was added to the plate at 50 µL per well, and incubated at 25°C for 1 hour.
6. Color development: After being washed four times, TMB color development solution was added to the plate at 50 µL per well, and developed color shielded from light at room temperature for 10 minutes.
7. Terminating: The stop solution was directly added to the plate at 50 µL per well to terminate the reaction.
8. Detection: After terminating the reaction, the microtiter plate was immediately put into the microplate reader. The OD value at 450 nm was measured, and the original data was saved for sorting. The results are shown in FIG. 8 and Table 5, showing that for the purified FabT, EC₅₀=0.05834; for FTF, EC₅₀=0.08869; for IgGT, EC₅₀=0.1041; and for FvT, EC₅₀=0.1661.

**Table 5 EC₅₀ of purified FabT, FTF, IgGT and FvT detected by ELISA**

| Antibody structure | FabT | FTF | IgGT | FvT |
|---|---|---|---|---|
| EC₅₀ | 0.05834 | 0.08869 | 0.1041 | 0.1661 |

### Example 4 Affinity Determination of Preferred Antibodies

The affinities of FabT and FTF were detected by Biacore T200 instrument. The specific protocols were as follows. Human PD-L1-His (constructed by GeneScience, SEQ ID NO: 11) and human VEGF-A-His (constructed by GeneScience, SEQ ID NO: 13) were coupled to CM5 biosensor chip (GE), and then the antibodies of different concentrations were flowed through the chip at a flow rate of 30 µL/min. The binding between the candidate antibody and antigen was performed with a binding time of 120 s and a dissociation time of 300 s. The kinetic fitting was performed using BIAevalution software (GE), and the results of affinity constants were obtained as shown in Table 6 and Table 7. The affinities of FabT, FTF, IgGT and FvT with PD-L1 were 6.16E-10 M, 1.04E-12 M, 6.37E-13 M and 3.37E-09 M, respectively; and the affinities of FabT, FTF, IgGT and FvT with VEGF-A were 1.75E-09 M, 2.00E-09 M, 2.77E-08 M and 2.40E-09 M, respectively.

For the bispecific antibodies that specifically bind to hPD-L1 and hVEGF-A in some specific embodiments, the bispecific antibody fusion protein with FabT structure has an amino acid sequence of heavy chain as shown in SEQ ID NO: 4, and an amino acid sequence of light chain as shown in SEQ ID NO: 8; the bispecific antibody fusion protein with FTF structure has an amino acid sequence of heavy chain as shown in SEQ ID NO: 5, and an amino acid sequence of light chain as shown in SEQ ID NO: 9; the bispecific antibody fusion protein with IgGT structure has an amino acid sequence of heavy chain as shown in SEQ ID NO: 6, and an amino acid sequence of light chain as shown in SEQ ID NO: 9; and the bispecific antibody fusion protein with FvT structure has an amino acid sequence of heavy chain as shown in SEQ ID NO: 7, and an amino acid sequence of light chain as shown in SEQ ID NO: 10.

**Table 6 Results of affinity detection of candidate bispecific molecules with PD-L1**

| Antibody | Ka (1/Ms) | Kd (1/s) | KD (M) | Rmax (RU) |
|---|---|---|---|---|
| FabT | 3.20E+05 | 1.97E-04 | 6.16E-10 | 20.7 |
| FTF | 1.83E+05 | 1.90E-07 | 1.04E-12 | 26.4 |
| IgGT | 2.34E+05 | 1.58E-07 | 6.37E-13 | 33.7 |
| FvT | 1.27E+05 | 4.29E-04 | 3.37E-09 | 18.6 |
| PD-L1 positive antibody | 3.64E+05 | 4.12E-07 | 1.13E-12 | 27.9 |

**Table 7 Results of affinity detection of candidate bispecific molecules with VEGF-A**

| Antibody | Ka (1/Ms) | Kd (1/s) | KD (M) | Rmax (RU) |
|---|---|---|---|---|
| FabT | 7.84E+05 | 1.37E-03 | 1.75E-09 | 14.6 |
| FTF | 3.11E+05 | 6.24E-04 | 2.00E-09 | 8.2 |
| IgGT | 3.66E+05 | 1.02E-03 | 2.77E-09 | 9.2 |
| FvT | 3.22E+05 | 7. 74E-04 | 2.40E-09 | 8.4 |
| Eylea positive fusion protein | 6.17E+05 | 9.32E-04 | 1.51E-09 | 13.2 |

### Example 5 Activity Determination of Preferred Antibodies on Blocking Cellular VEGFA Signaling Pathway

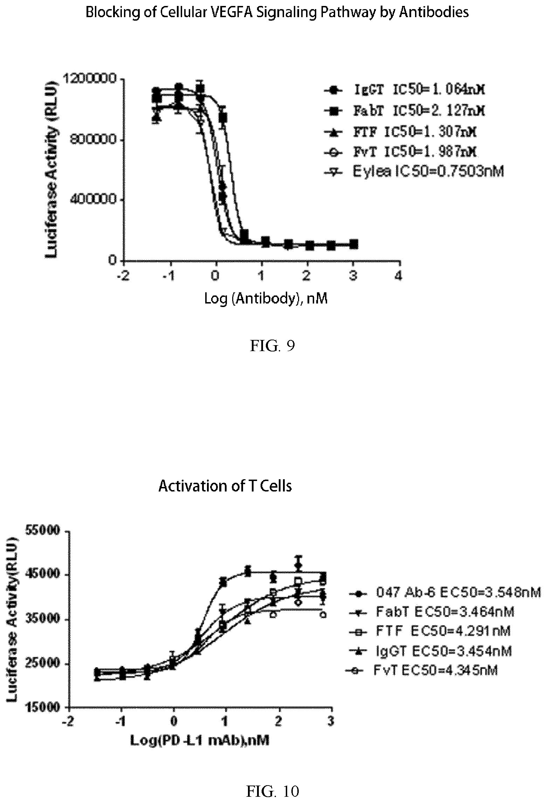

NFAT-RE-Luc2P-KDR-HEK293 cells (constructed by GeneScience, on the surface of which VEGFA's receptor KDR is expressed; when VEGFA binds to the KDR on the surface of HEK293 cells, the downstream signaling pathway is activated, and the binding of NFAT to NFAT *cis*-element leads to Luc2P expression to generate fluorescence) were seeded in a 96-well plate at 30,000/50µl/well. Each antibody was diluted in a 3-fold gradient with a total of 10 concentrations and a highest final concentration of 1 µM (stock concentration of 4 Mm, 25 µl/well); and the final concentration of VEGFA protein was 50 ng/ml (stock concentration of 200 ng/ml, 25 µl/well). Cells were lysed after 4h incubation in the incubator, and the reporter gene was detected. The results are shown in FIG. 9, which demonstrates that the blocking activity of the preferred bispecific molecules on the VEGFA signal is comparable to that of the control molecule Eylea.

### Example 6 Determination of T Cells Activation Activity of Preferred Antibodies

CHO-PDL1-CD3L cells (constructed by GeneScience, on the surface of which PDL1 and CD3L are expressed) were seeded in a 96-well plate at 40,000/well and placed in an incubator overnight to adhere. 047 Ab-6 control sample and four samples of bispecific antibody fusion proteins (FabT, FTF, IgGT and FvT) were subjected to test. The samples were diluted in a 3-fold gradient with a total of 10 concentrations and an initial concentration of 687.5 nM. After addition of diluted antibody, Jurkat-PD1-NFAT cells (constructed by GeneScience, on the surface of which PD1 is expressed; when the CD3L on the surface of CHO cells binds to Jurkat cells, the signal pathway in CHO cells is activated to therefore generate fluorescence, and when the PDL1 on the surface of CHO cells binds to the PD1 on the surface of Jurkat cells, NFAT signal pathway will be blocked and unable to generate fluorescence) were seeded in a culture plate at 100,000/well. The cells and antibodies were gently mixed and incubated for 6h, and then Bio-glo was added for detection. The results are shown in FIG. 10, which demonstrates that the cell activation activities of FabT, FTF, IgGT and FvT were slightly lower than that of the control antibody 047 Ab-6.

### Example 7 Drug Efficacy Determination of Preferred Antibodies in Animals

Model: C57BL/6 mice subcutaneously inoculated with MC38 cells (colorectal cancer cells) at 2×10⁵ cells/mouse

Administration: When the subcutaneous tumor volume reached about 100-150 mm³ mice were randomly divided into groups with 6 mice in each group, and administered intraperitoneally, twice a week for a total of 3 weeks: 047 Ab-6, 3 mg/kg; Eylea, 2 mg/kg; combination administration, 047 Ab-6 + Eylea, 3 mg/kg + 2 mg/kg, administered in the morning and evening; FabT, 2 mg/kg; FTF, 3.8 mg/kg; IgGT, 3.8 mg/ kg; and FvT, 3.8 mg/kg.

The results are shown in FIG. 10 and Table 8. The tumor inhibition rate of the control antibodies 047 Ab-6 and Eylea was 12% and 54%, respectively, and the tumor inhibition rate of the combination of 047 Ab-6 and Eylea reached 68%. The tumor inhibition rate of the bispecific antibody fusion proteins of the present invention was higher than that of combination of 047 Ab-6 and Eylea, and the tumor inhibition rates of FTF and IgGT reached 90% and 92%, respectively.

**Table 8 Tumor inhibition rate of candidate bispecific molecules**

| Drug | TGI (%) |
|---|---|
| 047 Ab-6 | 12 |
| Eylea | 54 |
| 047 Ab-6 + Eylea | 68 |
| FabT | 70 |
| FTF | 90 |
| IgGT | 92 |
| FvT | 81 |

The antibody fusion protein, preparation method thereof and application thereof provided by the present disclosure are described in detail above. Specific examples are given herein to illustrate the principle and embodiments of the present disclosure, and the illustration of these examples is only intended to facilitate understanding of the methods of the present disclosure and core concept thereof. It should be noted that, several improvements and modifications may be made by those skilled in the art to the present disclosure without departing from the principle of the present disclosure, and these improvements and modifications also fall within the protection scope of the claims thereof.

## Claims

1. An antibody fusion protein, comprising
(I) an antibody that specifically binds to a first antigen,
(II) a flexible peptide, and
(III) a fusion protein that specifically binds to a second antigen;
wherein the antibody that specifically binds to the first antigen comprises one or more fragments selected from light chain variable region, light chain constant region, heavy chain variable region, heavy chain constant region 1, heavy chain constant region 2, and heavy chain constant region 3.

2. The antibody fusion protein of claim 1, further comprising a hinge region.

3. The antibody fusion protein of claim 1 or 2, comprising
a1) light chain variable region and light chain constant region of the antibody that specifically binds to the first antigen, the flexible peptide and the fusion protein that specifically binds to the second antigen, represented as VL-CL-linker-Trap, and
b1) heavy chain variable region, heavy chain constant region 1 and partial hinge region of the antibody that specifically binds to the first antigen, the flexible peptide, and the fusion protein that specifically binds to the second antigen, represented as VH-CH1-Partial hinge-linker-Trap;
or comprising
a2) light chain of the antibody that specifically binds to the first antigen, and
b2) heavy chain variable region and heavy chain constant region 1 of the antibody that specifically binds to the first antigen, the flexible peptide, the fusion protein that specifically binds to the second antigen, heavy chain constant region 2 and heavy chain constant region 3, represented as VH-CH1-linker-Trap-CH2-CH3;
or comprising
a3) light chain of the antibody that specifically binds to the first antigen, and
b3) heavy chain variable region, heavy chain constant region 1, heavy chain constant region 2 and heavy chain constant region 3 of the antibody that specifically binds to the first antigen, the flexible peptide, and the fusion protein that specifically binds to the second antigen, represented as VH-CH1-CH2-CH3-linker-Trap;
or comprising
a4) light chain variable region of the antibody that specifically binds to the first antigen, the flexible peptide, the fusion protein that specifically binds to the second antigen, heavy chain constant region 2 and heavy chain constant region 3, represented as VL-linker-Trap-CH2-CH3, and
b4) heavy chain variable region of the antibody that specifically binds to the first antigen, the flexible peptide, the fusion protein that specifically binds to the second antigen, heavy chain constant region 2 and heavy chain constant region 3, represented as VH-linker-Trap-CH2-CH3.

4. The antibody fusion protein of any one of claims 1-3, wherein the flexible peptide comprises a sequence of (G4S)ₙ, wherein n is an integer greater than 0; preferably an integer of 1-10.

5. The antibody fusion protein of any one of claims 1-4, wherein the light chain constant region and the heavy chain constant region 1 form a heterodimer, and the terminal cysteine residue in the light chain constant region and the cysteine residue in the hinge region of heavy chain form a disulfide bond.

6. The antibody fusion protein of any one of claims 1-5, wherein the cysteine residues in the hinge regions of heavy chains form a disulfide bond.

7. The antibody fusion protein of any one of claims 1-6, wherein the domain of the heavy chain constant region 3 of first heavy chain and the domain of the heavy chain constant region 3 of second heavy chain are modified to a structure that facilitates the formation of the antibody fusion protein.

8. The antibody fusion protein of claim 7, wherein the modification comprises
c) modification to the domain of the heavy chain constant region 3 of the first heavy chain: in the interface between the domain of the heavy chain constant region 3 of the first heavy chain and the domain of the heavy chain constant region 3 of the second heavy chain of bivalent bispecific antibody, an amino acid residue in the domain of the heavy chain constant region 3 of the first heavy chain is replaced with an amino acid residue with a volume larger than the original amino acid residue to form a knob in the domain of the heavy chain constant region 3 of the first heavy chain, wherein the knob is capable of inserting into a hole of the domain of the heavy chain constant region 3 of the second heavy chain, and
d) modification to the domain of the heavy chain constant region 3 of the second heavy chain: in the interface between the domain of the heavy chain constant region 3 of the second heavy chain and the domain of the heavy chain constant region 3 of the first heavy chain of bivalent bispecific antibody, an amino acid residue in the domain of the heavy chain constant region 3 of the second heavy chain is replaced with an amino acid residue with a volume smaller than the original amino acid residue to form a hole in the domain of the heavy chain constant region 3 of the second heavy chain, wherein the hole is capable of holding the knob of the domain of the heavy chain constant region 3 of the first heavy chain.

9. The antibody fusion protein of claim 8, wherein in the heavy chain,
the amino acid residue with a volume larger than the original amino acid residue is selected from the group consisting of arginine, phenylalanine, tyrosine, and tryptophan; and
the amino acid residue with a volume smaller than the original amino acid residue is selected from the group consisting of alanine, serine, threonine, and valine.

10. The antibody fusion protein of any one of claims 1-9, comprising
(IV) the heavy chain with an amino acid sequence as shown in SEQ ID NO: 4 or SEQ ID NO: 5 or SEQ ID NO: 6 or SEQ ID NO: 7, and (V) the light chain with an amino acid sequence as shown in SEQ ID NO: 8 or SEQ ID NO: 9 or SEQ ID NO: 10;
or
(VI) an amino acid sequence derived from the amino acid sequence described in (IV) or (V) by substitution, deletion or addition of one or more amino acids, and functionally identical or similar to the amino acid sequence described in (IV) or (V);
or
(VII) an amino acid sequence with more than 90% homology with the sequence described in (IV) or (V);
or
(VIII) an amino acid sequence that has the same functional fragment or functional variant as the sequence described in (IV) or (V);
wherein the antibody fusion protein specifically binds to hPD-L1 and hVEGF-A; and
the first antigen is hPD-L1 and the second antigen is hVEGF-A.

11. The antibody fusion protein of claim 10, wherein the one or more amino acids is 2-10 amino acids.

12. The antibody fusion protein of any one of claims 1-11, comprising
(IX) a heavy chain with an amino acid sequence of SEQ ID NO: 4, and a light chain with an amino acid sequence of SEQ ID NO: 8; or
(X) a heavy chain with an amino acid sequence of SEQ ID NO: 5, and a light chain with an amino acid sequence of SEQ ID NO: 9; or
(XI) a heavy chain with an amino acid sequence of SEQ ID NO: 6, and a light chain with an amino acid sequence of SEQ ID NO: 9; or
(XII) a heavy chain with an amino acid sequence of SEQ ID NO: 7, and a light chain with an amino acid sequence of SEQ ID NO: 10.

13. A nucleic acid molecule encoding the antibody fusion protein of any one of claims 1-12, comprising
(XIII) a nucleic acid encoding the heavy chain variable region as shown in SEQ ID NO: 4 or SEQ ID NO: 5 or SEQ ID NO: 6 or SEQ ID NO: 7, and a nucleic acid encoding the light chain variable region as shown in SEQ ID NO: 8 or SEQ ID NO: 9 or SEQ ID NO: 10;
or
(XIV) a nucleic acid having complementary sequence of the heavy chain variable region as shown in SEQ ID NO: 4 or SEQ ID NO: 5 or SEQ ID NO: 6 or SEQ ID NO: 7, and a nucleic acid having complementary sequence of the light chain variable region as shown in SEQ ID NO: 8 or SEQ ID NO: 9 or SEQ ID NO: 10;
or
(XV) a nucleotide sequence encoding the same protein as the nucleotide sequence described in (XIII) or (XIV), but different from the nucleotide sequence described in (XIII) or (XIV) due to the degeneracy of genetic code;
or
(XVI) a sequence with more than 90% homology with the sequence described in (XIII) or (XIV) or (XV).

14. The nucleic acid molecule of claim 13, comprising a nucleotide sequence derived from the nucleotide sequence described in (XIII) or (XIV) or (XV) or (XVI) by substitution, deletion or addition of one or more nucleotide, and functionally identical or similar to the nucleotide sequence described in (XIII) or (XIV) or (XV) or (XVI), wherein the one or more amino acids is 2-10 amino acids.

15. An expression vector, comprising the nucleic acid molecule of claim 13 or 14, and a cell transformed with the expression vector.

16. A complex, comprising the antibody fusion protein of any one of claims 1-12 covalently linked to an isotope, an immunotoxin and/or a chemical drug.

17. A conjugate, formed by coupling the antibody fusion protein of any one of claims 1-12 and/or the complex of claim 16 with a solid medium or a semi-solid medium.

18. Use of the antibody fusion protein of any one of claims 1-12 and/or the complex of claim 16 and/or the conjugate of claim 17 for the manufacture of a medicament for the treatment of a disease and/or a composition for the diagnose of a disease;
wherein the disease is selected from the group consisting of breast cancer, lung cancer, gastric cancer, intestinal cancer, esophageal cancer, ovarian cancer, cervical cancer, kidney cancer, bladder cancer, pancreatic cancer, non-Hodgkin's lymphoma, chronic lymphoma leukemia, multiple myeloma, acute myeloid leukemia, acute lymphoma leukemia, glioma, melanoma, diabetic macular edema, and wet macular degeneration.

19. A pharmaceutical composition, comprising the antibody fusion protein of any one of claims 1-12 and/or the complex of claim 16 and/or the conjugate of claim 17.

20. A kit, comprising the antibody fusion protein of any one of claims 1-12 and/or the complex of claim 16 and/or the conjugate of claim 17.

21. A method for treating a disease with the antibody fusion protein of any one of claims 1-12 and/or the complex of claim 16 and/or the conjugate of claim 17, wherein the disease is selected from the group consisting of breast cancer, lung cancer, gastric cancer, intestinal cancer, esophageal cancer, ovarian cancer, cervical cancer, kidney cancer, bladder cancer, pancreatic cancer, non-Hodgkin's lymphoma, chronic lymphoma leukemia, multiple myeloma, acute myeloid leukemia, acute lymphoma leukemia, glioma, melanoma, diabetic macular edema, and wet macular degeneration.

22. A method for producing the antibody fusion protein of any one of claims 1-12, comprising transforming a host cell with the expression vector of claim 15, culturing the host cell under conditions that allow the synthesis of the antibody fusion protein, and recovering the antibody fusion protein from the culture.
